# EUROPEAN PATENT SPECIFICATION

(11) **EP 3 204 426 B1**
(45) Date of publication and mention of the grant of the patent: **25.11.2020**
(21) Application number: 15848940.1
(22) Date of filing: 09.10.2015
(51) Int. Cl.: A61K 35/16, C07K 16/34, A61P 31/14

(54) **COMPOSITIONS FOR USE IN TREATING VIRAL HEMORRHAGIC FEVER**
ZUSAMMENSETZUNGEN ZUR VERWENDUNG IN DER BEHANDLUNG VON VIRALEM HÄMORRHAGISCHEM FIEBER
COMPOSITIONS POUR L'UTILISATION DANS LE TRAITEMENT DE LA FIÈVRE HÉMORRAGIQUE VIRALE

(30) Priority: 10.10.2014 US 201462062803 P; 20.10.2014 US 201462066353 P
(43) Date of publication of application: 16.08.2017
(73) Proprietor: Cerus Corporation, Concord, CA 94520 (US)
(72) Inventor: CORASH, Laurence, Concord, CA 94520 (US)
(74) Representative: Mewburn Ellis LLP
(86) International application number: PCT/US2015/055007
(87) International publication number: WO 2016/057965

(56) References cited:
- US-A- 5 009 889
- US-B2- 7 901 673
- K Mupapa ET AL: "Treatment of Ebola hemorrhagic fever with blood transfusions from convalescent patients. International Scientific and Technical Committee", The Journal of Infectious Diseases, vol. 179 1 February 1999 (1999-02-01), pages S18-S23, XP055427030, Retrieved from the Internet: URL:https://academic.oup.com/jid/article-p df/179/Supplement_1/S18/17991476/179-Suppl ement_1-S18.pdf [retrieved on 2017-11-21]
- DATABASE BIOSIS [Online] BIOSCIENCES INFORMATION SERVICE, PHILADELPHIA, PA, US; November 2006 (2006-11), CAZENAVE JEAN-PIERRE ET AL: "Coagulation factors in therapeutic apheresis plasma held for 18 hours at ambient temperature prior to pathogen inactivation (INTERCEPT (TM)).", XP002779774, Database accession no. PREV200700257688 & BLOOD, vol. 108, no. 11, Part 1, November 2006 (2006-11), pages 280A-281A, 48TH ANNUAL MEETING OF THE AMERICAN-SOCIETY-OF-HEMATOLOGY; ORLANDO, FL, USA; DECEMBER 09 -12, 2006 ISSN: 0006-4971
- DATABASE BIOSIS [Online] BIOSCIENCES INFORMATION SERVICE, PHILADELPHIA, PA, US; November 2006 (2006-11), CAZENAVE JEAN-PIERRE ET AL: "Coagulation factor activity in therapeutic plasma prepared from whole blood with pathogen inactivation (INTERCEPT (TM)) treatment.", XP002779775, Database accession no. PREV200700245301 & BLOOD, vol. 108, no. 11, Part 2, November 2006 (2006-11), pages 117B-118B, 48TH ANNUAL MEETING OF THE AMERICAN-SOCIETY-OF-HEMATOLOGY; ORLANDO, FL, USA; DECEMBER 09 -12, 2006 ISSN: 0006-4971
- H.G. KLEIN ET AL: "Pathogen-reduction methods: advantages and limits", ISBT SCIENCE SERIES, vol. 4, 1 January 2009 (2009-01-01), pages 154-160, XP055464941,
- MUPAPA, K ET AL.: 'Treatment of Ebola Hemorrhagic Fever with Blood Transfusions from Convalescent Patients' J INFECT DIS. vol. 179, no. SUPPL, February 1999, pages 18 - 23, XP055427034
- GOWEN, B. ET AL.: 'Progress in the experimental therapy of severe arenaviral infections.' FUTURE MICROBIOL. vol. 6, no. 12, December 2011, pages 1429 - 1441, XP055427042

## Description

### TECHNICAL FIELD

The compositions and methods described herein generally relate to the treatment of viral hemorrhagic fever. More particularly, the disclosure relates to treating hemorrhagic fever virus infections and associated conditions with plasma preparations.

### BACKGROUND

Viral hemorrhagic fever is a clinical syndrome associated with significant mortality and morbidity. Hemorrhagic fever viruses generally belong to four viral families: the Filoviridae (*e.g.,* Ebola virus, Marburg virus), Arenaviridae (*e.g.,* Lassa virus, Junin virus), Bunyaviridae (*e.g.,* Rift Valley fever virus, Crimean-Congo hemorrhagic fever virus), and Flaviviridae (*e.g.,* dengue fever virus, Omsk hemorrhagic fever virus). These hemorrhagic fever viruses produce a wide range of disease severity and morbidities, with the most extreme conditions including circulatory instability, increased vascular permeability, and diffuse hemorrhage. Liver injury and hepatic dysfunction is another common occurrence in viral hemorrhagic fever. The underlying disease mechanisms associated with viral hemorrhagic fevers is complex, and may include among others, thrombocytopenia, disseminated intravascular coagulation (DIC), endothelial cell damage and dysfunction, activation of coagulation pathways, fibrin deposition and decreased levels of coagulation factors in blood.

Currently, no effective treatment has been identified for patients suffering from hemorrhagic fever virus infection. Interferon and ribavirin show limited effect against these agents. Evaluation of antibody therapies, including monoclonal and polyclonal antibody preparations, as well as passive transfer of blood or blood components from convalescent donors, has produced variable results in humans and animal models (Luke et al., 2010, Crit. Care Med. 38(4) Suppl.:e66-e73; Keller et al., 2000, Clin. Microbiol. Rev. 14(4):602-614; Enria et al., 2008, Antiviral Res. 78:132-139; Mikhailov et al, 1994, Voprosi Virusologii, 39P82-84; Jahrling et al., 1996, Arch Virol, US, 135-140; Jahrling et al., 1999, J Infect Dis, 179 (Suppl 1), S224-234; Kudoyarova-Zubavichene et al., 1999, J Infect Dis, 179 (Suppl 1), S218-223); Jahrling et al., 2007, J. Infect. Dis. 196(Suppl. 2):S400-S403; Maruyama et al. J. Infect. Dis. 179 (suppl 1), S235, 1999; Maruyama et al. J. Virol. 73, 6024, 1999; Parren et al. J. Virol 76, 6408, 2002); Mupapa, et al., 1999, J Infect Dis 179 Suppl 1: S18-23), with none approved for use in humans. In the case of passive transfer of blood products from convalescent donors, an additional risk of transfusion transmitted infection due to other pathogens presents further concerns.

Mupapa, et al., 1999, J Infect Dis 179 Suppl 1: S18-23 describes the treatment of Ebola hemorrhagic fever using transfusion of whole blood obtained from convalescent Ebola virus patients. Cazenave et al. Blood, 108 (11), Part 1: 280A-281A; and Part 2, 117B-118B describes apheresis plasma processed with the INTERCEPT system for pathogen inactivation retained coagulation factor activity levels in line with French national standards for therapeutic frozen plasma. The authors measured IgG, IgA and IgM proteins (among other components of plasma).

In the absence of specific pharmaceutical or biologic therapies, management of such viral infections is primarily supportive. Therefore, a clear unmet need exists for a safe and effective treatment for patients with viral hemorrhagic fever.

### SUMMARY

The present disclosure provides compositions and their use in methods for the treatment of viral hemorrhagic fever. The compositions are useful for treating hemorrhagic fever virus infections and conditions associated with such infections, and include for example, the use of plasma obtained from one or more donors previously infected with the same type of hemorrhagic fever virus (*e.g.,* immune plasma, convalescent plasma) as the subject with viral hemorrhagic fever in need of treatment. The present disclosure provides certain treatment advantages, for example by utilizing in certain embodiments, both plasma obtained from donor(s) previously infected with the hemorrhagic fever virus and plasma obtained from donor(s) not previously infected with the hemorrhagic fever virus (*e.g.,* non-immune plasma). While dilution (*e.g.,* reduced volume) of convalescent plasma may seem on first impression to be less desirable (*e.g.,* less effective) for treatment use due to corresponding dilution of immune components therein, the inclusion of non-immune plasma counterintuitively provides an improved treatment for viral hemorrhagic fever infection, as well as extending the supply of convalescent plasma.

In one aspect, the present invention provides a first plasma component for use in a method of treating a subject suffering from a hemorrhagic fever virus infection in combination with a second plasma component, said method comprising administering to the subject a therapeutically effective amount of said first and said second plasma component, wherein the first plasma component is obtained from one or more plasma donors previously infected with the same type of hemorrhagic fever virus and the second plasma component is obtained from one or more plasma donors not previously infected with the same type of hemorrhagic fever virus; wherein the first plasma component or donor plasma obtained for the first plasma component is treated with a pathogen inactivation compound to inactivate pathogens; wherein the hemorrhagic fever virus is a bunyavirus, arenavirus, flavivirus or filovirus; and wherein optionally the filovirus is an Ebola virus or a Marburg virus.

In a further aspect, the present invention provides a first and a second plasma component for use in combination in a method of treating a subject suffering from a hemorrhagic fever virus infection, said method comprising administering to the subject a therapeutically effective amount of said first and said second plasma component, wherein the first plasma component is obtained from one or more plasma donors previously infected with the same type of hemorrhagic fever virus and the second plasma component is obtained from one or more plasma donors not previously infected with the same type of hemorrhagic fever virus; wherein the first plasma component or donor plasma obtained for the first plasma component is treated with a pathogen inactivation compound to inactivate pathogens; wherein the hemorrhagic fever virus is a bunyavirus, arenavirus, flavivirus or filovirus; and wherein optionally the filovirus is an Ebola virus or a Marburg virus. In some embodiments, the hemorrhagic fever virus is a filovirus. In some embodiments, the filovirus is an Ebola virus (*e.g.,* Zaire ebolavirus, Bundibugyo ebolavirus, Reston ebolavirus, Sudan ebolavirus, Taï Forest ebolavirus) or a Marburg virus. In some embodiments, the hemorrhagic fever virus is an arenavirus. In some embodiments, the arenavirus is a Lassa fever virus, a Lujo virus, a Argentine hemorrhagic fever virus (*e.g.,* Junin virus), a Bolivian hemorrhagic fever virus (*e.g.,* Machupo virus), a Brazilian hemorrhagic fever virus (*e.g.,* Sabia virus), a Venezuelan hemorrhagic fever virus (*e.g.,* Guanarito virus) or Chapare hemorrhagic fever virus. In some embodiments, the hemorrhagic fever virus is a bunyavirus. In some embodiments, the bunyavirus is a Rift Valley fever virus, Crimean-Congo hemorrhagic fever virus, a Garissa virus, a Ilesha virus or a hantavirus (*e.g.,* Hantaan virus, Dobrava virus, Saaremaa virus, Seoul virus, Puumala virus). In some embodiments, the hemorrhagic fever virus is a flavivirus. In some embodiments, the flavivirus is a dengue fever virus, a yellow fever virus, Alkhurma homorrhagic fever virus, or a tick-borne encephalitis (*e.g.,* Omsk hemorrhagic fever virus, Kyasanur Forest disease virus). In some embodiments, the hemorrhagic fever virus is a rhabdovirus (*e.g.,* Bas-Congo virus, BASV).

In some embodiments, the first plasma component and the second plasma component are each obtained from one or more human donors. In some embodiments, the first plasma component is obtained from one donor. In some embodiments, the first plasma component is obtained from at least two donors. In some embodiments, the first plasma component is obtained from 2-12 donors. In some embodiments, the first plasma component is obtained from 2 donors, 3 donors, 4 donors, 5 donors, 6 donors, 7 donors, 8 donors, 9 donors, 10 donors, 11 donors or 12 donors. In some embodiments, the second plasma component is obtained from one donor. In some embodiments, the second plasma component is obtained from at least two donors. In some embodiments, the second plasma component is obtained from 2-12 donors. In some embodiments, the second plasma component is obtained from 2 donors, 3 donors, 4 donors, 5 donors, 6 donors, 7 donors, 8 donors, 9 donors, 10 donors, 11 donors or 12 donors. In some embodiments, the first plasma component, the second plasma component, or both the first plasma component and second plasma component comprises donor plasma only of the same ABO blood group as the subject. In some embodiments, the first plasma component comprises donor plasma of more than one ABO blood group. In some embodiments, the first plasma component comprises donor plasma of blood group A and blood group B. In some embodiments, the first plasma component is obtained from at least 3 donors and wherein the first plasma component comprises donor plasma of blood group A, blood group B and blood group AB. In some embodiments, the first plasma component does not contain donor plasma of blood group O. In some embodiments, the second plasma component comprises donor plasma of more than one ABO blood group. In some embodiments, the second plasma component comprises donor plasma of blood group A and blood group B. In some embodiments, the second plasma component is obtained from at least 3 donors and wherein the second plasma component comprises donor plasma of blood group A, blood group B and blood group AB. In some embodiments, the second plasma component does not contain donor plasma of blood group O.

In some embodiments, the first plasma component is obtained only from donors with no clinical symptoms of infection with the hemorrhagic fever virus at the time of donating the plasma for said first plasma component. In some embodiments, the first plasma component is obtained only from donors with no detectable levels of the hemorrhagic fever virus in their (*i.e.,* the donor's) blood at the time of donating the plasma for said first plasma component. In some embodiments, no detectable levels of the hemorrhagic fever virus in their blood is no detectable levels of the virus as determined by nucleic acid testing. In some embodiments, the virus is below the limit of detection in the donor's blood by nucleic acid testing. In some embodiments, nucleic acid testing is by PCR (*e.g.,* RT-PCR).

In some embodiments of the invention, the plasma (*e.g.,* plasma administered) comprises about 10-90% of the total volume as the first plasma component and the remainder of the total volume as the second plasma component. In some embodiments, the plasma (*e.g.,* plasma administered) comprises about 10-50% of the total volume as the first plasma component and the remainder of the total volume as the second plasma component. In some embodiments, the plasma (*e.g.,* plasma administered) comprises about 10-80%, about 10-70%, about 10-60%, about 10-40%, about 10-30% or about 10-20% of the total volume as the first plasma component and the remainder of the total volume as the second plasma component. In some embodiments, the plasma (*e.g.,* plasma administered) comprises about 10%, about 20%, about 30%, about 40%, about 50%, about 60%, about 70%, about 80% or about 90% of the total volume as the first plasma component and the remainder of the total volume as the second plasma component. In some embodiments, the first plasma component comprises about 10-90% of the total plasma volume administered and the second plasma component comprises the remainder of the total plasma volume administered. In some embodiments, the first plasma component comprises about 10-50% of the total plasma volume administered and the second plasma component comprises the remainder of the total plasma volume administered. In some embodiments, the first plasma component comprises about 10-80%, about 10-70%, about 10-60%, about 10-40%, about 10-30% or about 10-20% of the total plasma volume administered and the second plasma component comprises the remainder of the total plasma volume administered. In some embodiments, the first plasma component comprises about 10%, about 20%, about 30%, about 40%, about 50%, about 60%, about 70%, about 80% or about 90% of the total plasma volume administered and the second plasma component comprises the remainder of the total plasma volume administered. In some embodiments, the plasma (*e.g.,* plasma administered) comprises a volume ratio of about 1:1 for the second plasma component and the first plasma component. In some embodiments, the plasma (*e.g.,* plasma administered) comprises a volume ratio of about 2:1 for the second plasma component and the first plasma component. In some embodiments, the plasma (*e.g.,* plasma administered) comprises a volume ratio of at least about 3:1, at least about 4:1, at least about 5:1, at least about 6:1, at least about 7:1, at least about 8:1, at least about 9:1 or at least about 10:1 for the second plasma component and the first plasma component. In some embodiments, the plasma (*e.g.,* plasma administered) comprises less than about 50% of the total volume as the first plasma component.

In some embodiments of the invention, the first plasma component, the second plasma component or both the first plasma component and second plasma component are frozen for storage and thawed prior to administration of the plasma to the subject. In some embodiments of the aforementioned methods, the first plasma component, the second plasma component, or both the first plasma component and second plasma component are lyophilized for storage and reconstituted prior to administration of the plasma to the subject. In some embodiments, the first plasma component, the second plasma component, or both the first plasma component and second plasma component are obtained from the one or more donors by apheresis.

In some embodiments of the invention, the first plasma component and the second plasma component are administered separately to the subject. In some embodiments, the first plasma component and the second plasma component are administered sequentially to the subject. In some embodiments of the invention, the first plasma component and the second plasma component are administered within about 72 hours, within about 48 hours, within about 24 hours, within about 15 hours, within about 8 hours, within about 4 hours, within about 2 hours, or within about 1 hour of each other. In some embodiments, the second plasma component is administered before the first plasma component. In some embodiments, the second plasma component is administered as a plasma exchange (*e.g.,* plasmapheresis, therapeutic plasma exchange). In some embodiments, the plasma exchange comprises about 1.0 to 1.5 blood volumes of plasma (*e.g.,* total plasma volume of the subject). In some embodiments, the plasma exchange comprises a total (*e.g.,* 100%) plasma volume. In some embodiments, the plasma exchange volume is adjusted (*e.g.,* increased) relative to a dilution of the plasma from a pathogen inactivation process (*e.g.,* treated with a pathogen inactivation compound). In some embodiments, the first plasma component and the second plasma component are administered to the subject at about the same time. In some embodiments, the first plasma component and the second plasma component are mixed prior to or during administration to the subject.

In some embodiments of the invention, the plasma is administered to the subject in a volume (*e.g.,* total volume, total volume of one or more units) of about 200-2500 mL, about 200-2000mL, about 200-1500 mL, about 200-1000 mL or about 200-500 mL. In some embodiments, the plasma is administered to the subject in a volume of about 300-2500 mL. In some embodiments, the plasma is administered to the subject in a volume of about 300-2000 mL. In some embodiments, the plasma is administered to the subject in a volume of about 300-1500 mL. In some embodiments, the plasma is administered to the subject in a volume of about 300-1200 mL, about 300-1000 mL, about 300-800 mL, about 300-700 mL, about 300-600 mL, about 300-500 mL or about 300-400 mL. In some embodiments, the plasma is administered to the subject in a volume of about 400-500 mL or 400-600 mL. In some embodiments, the plasma is administered to the subject in volume of about 15-20 mL/kg body weight of the subject (*e.g.,* weight adjusted dosing). In some embodiments, the plasma is administered to the subject in volume of about 40-60 mL/kg body weight of the subject. In some embodiments, the plasma is administered to the subject in volume of about 10 mL/kg, about 15 mL/kg, about 20 mL/kg, about 25 mL/kg, about 30 mL/kg, about 40 mL/kg, about 50 mL/kg or about 60 mL/kg body weight of the subject. In some embodiments, the plasma is administered by infusion. In some embodiments, the plasma is administered to the subject as one or more infusions, two or more infusions, three or more infusions or four or more infusions.

In some embodiments of the invention, the level of viral infection (*e.g.,* viral load, virus titer) in the subject is reduced. In some embodiments, the level of viral infection is reduced at least 1 log, at least 2 logs, at least 3 logs, at least 4 logs, at least 5 logs or at least 6 or more logs. In some embodiments, the level of viral infection is reduced to no detectable hemorrhagic fever virus in blood of the subject. In some embodiments, no detectable hemorrhagic fever virus in blood of the subject is no detectable hemorrhagic fever virus as determined by nucleic acid testing. In some embodiments, the viral infection is reduced in the subject's blood below the limit of detection by nucleic acid testing. In some embodiments, nucleic acid testing is by PCR (*e.g.,* RT-PCR). In some embodiments, the mortality associated with the virus infection is reduced. In some embodiments, the number of co-morbidities or severity of morbidity (*e.g.,* severity of one or more morbidities) is reduced (*e.g.,* decreased, ameliorated). In some embodiments, the absence of clinical symptoms indicative of viral hemorrhagic fever disease is achieved. In some embodiments, coagulopathy associated with the virus infection is reduced. In some embodiments, endothelial cell dysfunction (*e.g.,* damage) associated with the virus infection is decreased. In some embodiments, endothelial cell function (*e.g.,* associated with damage from the virus infection) is restored (*e.g.,* partially restored). In some embodiments, endothelial barrier function is enhanced. In some embodiments, time of hospitalization is reduced. In some embodiments, time spent in ICU (*e.g.,* intensive care, an intensive care unit) is reduced. In some embodiments, total duration and/or frequency of dialysis is decreased. In some embodiments, time on assisted ventilation is decreased. In some embodiments, incidence of organ failure is reduced. In some embodiments, multifocal necrosis (*e.g.,* multifocal hepatic necrosis) associated with virus infection is reduced.

In some embodiments of the invention, the first plasma component is a concentrated (*e.g.,* purified) immunoglobulin preparation. In some embodiments of the invention, the second plasma component is a plasma cryoprecipitate. In some embodiments of the invention, the method further comprises treating the plasma with a pathogen inactivation compound to inactivate pathogens, if present. In some embodiments, the method further comprises treating the first plasma component and/or the second plasma component with a pathogen inactivation compound to inactivate pathogens, if present. In some embodiments, the method further comprises treating the donor plasma obtained for the first plasma component and/or the donor plasma obtained for the second plasma component with a pathogen inactivation compound to inactivate pathogens, if present. In some embodiments, treatment with a pathogen inactivation compound is a photochemical treatment. In some embodiments, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some embodiments, the pathogen inactivation compound is a psoralen. In some embodiments, the pathogen inactivation compound is amotosalen. In some embodiments, the first and second plasma components are treated with the pathogen inactivation compound. In some embodiments, the level of binding activity of hemorrhagic fever virus specific antibody in the first plasma component after treatment with the pathogen inactivation compound is at least 80% of the level of binding activity of hemorrhagic fever virus specific antibody before treatment with the pathogen inactivation compound. In some embodiments, the donor plasma obtained for the first and second plasma components is treated with the pathogen inactivation compound. In some embodiments, the level of binding activity of hemorrhagic fever virus specific antibody in the first plasma component after treatment with the pathogen inactivation compound is at least 50%, 60%, 70%, 90% or 95% of the level of binding activity of hemorrhagic fever virus specific antibody before treatment with the pathogen inactivation compound. In some embodiments, the donor plasma obtained for the first and second plasma components is treated with the pathogen inactivation compound. In some embodiments, the level of binding activity of hemorrhagic fever virus specific antibody in the donor plasma obtained for the first plasma component, after treatment with the pathogen inactivation compound, is at least 80% of the level of binding activity of hemorrhagic fever virus specific antibody in the donor plasma before treatment with the pathogen inactivation compound. In some embodiments, the level of binding activity of hemorrhagic fever virus specific antibody in the donor plasma obtained for the first plasma component, after treatment with the pathogen inactivation compound, is at least 50%, 60%, 70%, 90% or 95% of the level of binding activity of hemorrhagic fever virus specific antibody in the donor plasma before treatment with the pathogen inactivation compound. In some embodiments of the aforementioned methods, the method further comprises administering an antiviral agent.

The present disclosure further provides a method of treating a condition associated with a hemorrhagic fever virus infection (*e.g*., viral hemorrhagic fever) in a subject, comprising administering a plasma (*e.g*., plasma preparation, plasma therapeutic) in an amount effective to treat (*e.g*., reduce, decrease, prevent, suppress, ameliorate) coagulopathy or endothelial cell dysfunction, wherein the plasma comprises donor plasma obtained from one or more donors (*e.g*., human donors) previously infected with or immunized against the same type (*e.g.,* same genus, same species, same subtype) of hemorrhagic fever virus (*i.e.,* same type of hemorrhagic fever virus as the subject), and wherein the plasma or donor plasma is treated with a pathogen inactivation compound to inactivate pathogens, if present. In some instances, the method of treating a condition associated with a hemorrhagic fever virus infection in a subject comprises administering a plasma in an amount effective to treat coagulopathy and endothelial dysfunction. In some instances, the donor plasma obtained from one or more donors previously infected with or immunized against the same type of hemorrhagic fever virus is donor plasma obtained from one or more plasma donors previously infected with the same type of hemorrhagic fever virus. In some instances, the hemorrhagic fever virus is a bunyavirus, arenavirus, flavivirus or filovirus. In some instances, the hemorrhagic fever virus is a filovirus. In some instances, the filovirus is an Ebola virus (*e.g*., Zaire ebolavirus, Bundibugyo ebolavirus, Reston ebolavirus, Sudan ebolavirus, Taï Forest ebolavirus) or a Marburg virus. In some instances, the hemorrhagic fever virus is an arenavirus. In some instances, the arenavirus is a Lassa fever virus, a Lujo virus, a Argentine hemorrhagic fever virus (*e.g.,* Junin virus), a Bolivian hemorrhagic fever virus *(e.g.,* Machupo virus), a Brazilian hemorrhagic fever virus *(e.g.,* Sabia virus), a Venezuelan hemorrhagic fever virus (*e.g.,* Guanarito virus) or Chapare hemorrhagic fever virus. In some embodiments, the hemorrhagic fever virus is a bunyavirus. In some embodiments, the bunyavirus is a Rift Valley fever virus, Crimean-Congo hemorrhagic fever virus, a Garissa virus, a Ilesha virus or a hantavirus (*e.g.,* Hantaan virus, Dobrava virus, Saaremaa virus, Seoul virus, Puumala virus). In some embodiments, the hemorrhagic fever virus is a flavivirus. In some embodiments, the flavivirus is a dengue fever virus, a yellow fever virus, Alkhurma homorrhagic fever virus, or a tick-borne encephalitis (*e.g.,* Omsk hemorrhagic fever virus, Kyasanur Forest disease virus). In some embodiments, the hemorrhagic fever virus is a rhabdovirus (*e.g.,* Bas-Congo virus, BASV).

In some instances of the aforementioned methods, the subject is a human subject. In some instances of the aforementioned methods, the donor plasma is obtained from one donor. In some instances, the donor plasma is of the same ABO blood group as the subject. In some instances of the aforementioned methods, the donor plasma is obtained from at least two donors. In some instances, the donor plasma is obtained from 2-12 donors. In some instances, the donor plasma is obtained from 2 donors, 3 donors, 4 donors, 5 donors, 6 donors, 7 donors, 8 donors, 9 donors, 10 donors, 11 donors or 12 donors. In some instances, the plasma comprises donor plasma only of the same ABO blood group as the subject. In some embodiments, the plasma comprises donor plasma of more than one ABO blood group. In some instances, the plasma comprises donor plasma of blood group A and blood group B. In some embodiments, the plasma is obtained from at least 3 donors and wherein the plasma comprises donor plasma of blood group A, blood group B and blood group AB. In some instances, the plasma does not contain donor plasma of blood group O.

In some instances of the aforementioned methods, the donor plasma obtained from one or more donors previously infected with or immunized against the hemorrhagic fever virus is obtained only from donors with no clinical symptoms of infection (*e.g.,* infection with the hemorrhagic fever virus) with the virus at the time of donating plasma. In some instances of the aforementioned methods, the donor plasma obtained from one or more donors previously infected with or immunized against the hemorrhagic fever virus is obtained only from donors that have no detectable levels of the virus in their *(i.e.,* the donor's) blood at the time of donating plasma. In some instances, no detectable levels of the virus in their blood is no detectable levels of the virus as determined by nucleic acid testing. In some instances, the virus is below the limit of detection in the donor's blood by nucleic acid testing. In some instances, nucleic acid testing is by PCR (*e.g.,* RT-PCR).

In some instances of the aforementioned methods, the plasma or donor plasma is frozen for storage and thawed prior to administration of the plasma to the subject. In some instances of the aforementioned methods, the plasma or donor plasma is lyophilized for storage and reconstituted prior to administration of the plasma to the subject. In some instances of the aforementioned methods, the plasma or donor plasma is a concentrated (*e.g.,* purified) immunoglobulin preparation. In some instances of the aforementioned methods, the donor plasma is obtained from the one or more donors by apheresis. In some instances of the aforementioned methods, the plasma or donor plasma is treated with a pathogen inactivation compound to inactivate pathogens, if present. In some instances, treatment with a pathogen inactivation compound is a photochemical treatment. In some instances, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some instances, the pathogen inactivation compound is a psoralen. In some instances, the pathogen inactivation compound is amotosalen.

In some embodiments of the invention, the donor plasma obtained from one or more donors previously infected with or immunized against the hemorrhagic fever virus is a first donor plasma and the method further comprises administering a second donor plasma obtained from one or more donors not previously infected with or immunized against the same type of hemorrhagic fever virus (*i.e.,* same type of hemorrhagic fever virus as the subject), and wherein the second donor plasma is treated with a pathogen inactivation compound to inactivate pathogens, if present. In some embodiments, treatment with a pathogen inactivation compound is a photochemical treatment. In some embodiments, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some embodiments, the pathogen inactivation compound is a psoralen. In some embodiments, the pathogen inactivation compound is amotosalen.

In some embodiments of the invention, the donor plasma obtained from one or more donors previously infected with or immunized against the hemorrhagic fever virus is a first donor plasma and the plasma further comprises a second donor plasma obtained from one or more donors not previously infected with or immunized against the same type of hemorrhagic fever virus (*i.e.,* same type of hemorrhagic fever virus as the subject), and wherein the second donor plasma is optionally treated with a pathogen inactivation compound to inactivate pathogens, if present. In some embodiments, treatment with a pathogen inactivation compound is a photochemical treatment. In some embodiments, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some embodiments, the pathogen inactivation compound is a psoralen. In some embodiments, the pathogen inactivation compound is amotosalen.

In some embodiments of the invention, the second donor plasma is obtained from one donor. In some embodiments, the second donor plasma is of the same ABO blood group as the subject. In some embodiments of the aforementioned methods, the second donor plasma is obtained from at least two donors. In some embodiments, the second donor plasma is obtained from 2-12 donors. In some embodiments, the second donor plasma is obtained from 2 donors, 3 donors, 4 donors, 5 donors, 6 donors, 7 donors, 8 donors, 9 donors, 10 donors, 11 donors or 12 donors. In some embodiments, the second donor plasma comprises plasma only of the same ABO blood group as the subject. In some embodiments, the second donor plasma comprises plasma of more than one ABO blood group. In some embodiments, the second donor plasma comprises plasma of blood group A and blood group B. In some embodiments, the second donor plasma is obtained from at least 3 donors and wherein the second donor plasma comprises plasma of blood group A, blood group B and blood group AB. In some embodiments, the second donor plasma does not contain plasma of blood group O.

In some embodiments of the invention, the second donor plasma is frozen for storage and thawed prior to administration of the plasma (*e.g.,* the second donor plasma) to the subject. In some embodiments of the invention, the second donor plasma is lyophilized for storage and reconstituted prior to administration of the plasma (*e.g.,* the second donor plasma) to the subject. In some embodiments of the invention, the second donor plasma is a plasma cryoprecipitate. In some embodiments of the invention, the plasma (*e.g.,* plasma administered) comprises about 10-90% of the total volume as the first donor plasma and the remainder of the total volume as the second donor plasma. In some embodiments, the plasma (*e.g.,* plasma administered) comprises about 10-50% of the total volume as the first donor and the remainder of the total volume as the second donor plasma. In some embodiments, the plasma (*e.g.,* plasma administered) comprises about 10-80%, about 10-70%, about 10-60%, about 10-40%, about 10-30% or about 10-20% of the total volume as the first donor plasma and the remainder of the total volume as the second donor plasma. In some embodiments, the plasma (*e.g.,* plasma administered) comprises about 10%, about 20%, about 30%, about 40%, about 50%, about 60%, about 70%, about 80% or about 90% of the total volume as the first donor plasma and the remainder of the total volume as the second donor plasma. In some embodiments, the first donor plasma comprises about 10-90% of the total plasma volume administered and the second donor plasma comprises the remainder of the total plasma volume administered. In some embodiments, the first donor plasma comprises about 10-50% of the total plasma volume administered and the second donor plasma comprises the remainder of the total plasma volume administered. In some embodiments, the first donor plasma comprises about 10-80%, about 10-70%, about 10-60%, about 10-40%, about 10-30% or about 10-20% of the total plasma volume administered and the second donor plasma comprises the remainder of the total plasma volume administered. In some embodiments, the first donor plasma comprises about 10%, about 20%, about 30%, about 40%, about 50%, about 60%, about 70%, about 80% or about 90% of the total plasma volume administered and the second donor plasma comprises the remainder of the total plasma volume administered. In some embodiments, the plasma (*e.g.,* plasma administered) comprises a volume ratio of about 1:1 for the second donor plasma and the first donor plasma. In some embodiments, the plasma (*e.g.,* plasma administered) comprises a volume ratio of about 2:1 for the second donor plasma and the first donor plasma. In some embodiments, the plasma (*e.g.,* plasma administered) comprises a volume ratio of at least about 3:1, at least about 4:1, at least about 5:1, at least about 6:1, at least about 7:1, at least about 8:1, at least about 9:1 or at least about 10:1 for the second donor plasma and the first donor plasma. In some embodiments, the plasma (*e.g.,* plasma administered) comprises less than about 50% of the total volume as the first donor plasma.

In some embodiments of the invention, the first donor plasma and the second donor plasma are administered separately to the subject. In some embodiments, the first donor plasma and the second donor plasma administered sequentially to the subject. In some embodiments, the first donor plasma and the second donor plasma are administered within about 72 hours, within about 48 hours, within about 24 hours, within about 15 hours, within about 8 hours, within about 4 hours, within about 2 hours, or within about 1 hour of each other. In some embodiments, the second donor plasma is administered before the first donor plasma. In some embodiments, the second donor plasma is administered as a plasma exchange (*e.g.,* plasmapheresis, therapeutic plasma exchange). In some embodiments, the plasma exchange comprises about 1.0 to 1.5 blood volumes of plasma (*e.g.,* total plasma volume of the subject). In some embodiments, the plasma exchange comprises a total (*e.g.,* 100%) plasma volume. In some embodiments, the plasma exchange volume is adjusted (*e.g.,* increased) relative to a dilution of the plasma from a pathogen inactivation process *(e.g.,* treated with a pathogen inactivation compound). In some embodiments, the first donor plasma and the second donor plasma are administered to the subject at about the same time. In some embodiments, the first donor plasma and the second donor plasma are mixed prior to or during administration to the subject.

In some embodiments of the invention, the plasma is administered to the subject in a volume (*e.g.,* total volume, total volume of one or more units) of about 200-2500 mL, about 200-2000mL, about 200-1500 mL, about 200-1000 mL or about 200-500 mL. In some embodiments, the plasma is administered to the subject in a volume of about 300-2500 mL. In some embodiments, the plasma is administered to the subject in a volume of about 300-2000 mL. In some embodiments, the plasma is administered to the subject in a volume of about 300-1500 mL. In some embodiments, the plasma is administered to the subject in a volume of about 300-1200 mL, about 300-1000 mL, about 300-800 mL, about 300-700 mL, about 300-600 mL, about 300-500 mL or about 300-400 mL. In some embodiments, the plasma is administered to the subject in a volume of about 400-500 mL or 400-600 mL. In some embodiments, the plasma is administered to the subject in volume of about 15-20 mL/kg body weight of the subject (*e.g.,* weight adjusted dosing). In some embodiments, the plasma is administered to the subject in volume of about 40-60 mL/kg body weight of the subject. In some embodiments, the plasma is administered to the subject in volume of about 10 mL/kg, about 15 mL/kg, about 20 mL/kg, about 25 mL/kg, about 30 mL/kg, about 40 mL/kg, about 50 mL/kg or about 60 mL/kg body weight of the subject. In some embodiments, the plasma is administered by infusion. In some embodiments, the plasma is administered to the subject as one or more infusions, two or more infusions, three or more infusions or four or more infusions.

In some embodiments of the invention, the level of viral infection (*e.g.,* viral load, virus titer) in the subject is reduced. In some embodiments, the level of viral infection is reduced to no detectable hemorrhagic fever virus in blood of the subject. In some embodiments, no detectable hemorrhagic fever virus in blood of the subject is no detectable hemorrhagic fever virus as determined by nucleic acid testing. In some embodiments, the viral infection is reduced in the subject's blood below the limit of detection by nucleic acid testing. In some embodiments, nucleic acid testing is by PCR (*e.g.,* RT-PCR). In some embodiments, the mortality associated with the virus infection is reduced. In some embodiments, the number of co-morbidities or severity of morbidity (*e.g.,* severity of one or more morbidities) is reduced (*e.g.,* decreased, ameliorated). In some embodiments, the absence of clinical symptoms indicative of viral hemorrhagic fever disease is achieved. In some embodiments, coagulopathy is reduced. In some embodiments, coagulopathy (*e.g.,* the degree of coagulopathy) is determined by one or more analytical measures known in the art for assessing coagulopathy, such as for example, thrombin generation, prothrombin time (PT), international normalized ratio (INR), activated partial thromboplastin time (aPTT), fibrinogen and platelet count. In some embodiments, endothelial cell dysfunction (*e.g.,* damage) is decreased. In some embodiments, endothelial cell function (*e.g.,* associated with damage from the virus infection) is restored (*e.g.,* partially restored). In some embodiments, endothelial cell function or dysfunction is determined by measurement of one or more biomarkers selected from the group consisting of von Willebrand factor (vWF), ADAMTS13, angiopoietins (Ang)-1 and -2, endocan, selectins (*e.g.,* E-, P-, L-), endothelial leukocyte adhesion molecule (E-selectin or ELAM-1), endothelin (ET-1), endothelin precursor peptide proET-1, VEGF, soluble VEGF-receptor-1 (Flt-1), PDGF, plasminogen activator inhibitor (PAI-1), urokinase PA (uPA) and fibrin degradation products (*e.g.,* X and Y fragments, D-dimers, D and E fragments, Bβ15-42). In some embodiments, the endothelial barrier function is enhanced. In some embodiments, time of hospitalization is reduced. In some embodiments, time spent in ICU is reduced. In some embodiments, total duration or frequency of dialysis is decreased. In some embodiments, time on assisted ventilation is decreased. In some embodiments, incidence of organ failure is reduced. In some embodiments, multifocal necrosis (*e.g.,* multifocal hepatic necrosis) associated with virus infection is reduced.

In some embodiments of the invention, the level of binding activity of hemorrhagic fever virus specific antibody in the donor plasma obtained from one or more donors previously infected with or immunized against the hemorrhagic fever virus, after treatment with the pathogen inactivation compound, is at least 80% of the level of binding activity of hemorrhagic fever virus specific antibody before treatment with the pathogen inactivation compound. In some embodiments of the invention, the level of binding activity of hemorrhagic fever virus specific antibody in the donor plasma obtained from one or more donors previously infected with or immunized against the hemorrhagic fever virus, after treatment with the pathogen inactivation compound, is at least 50%, 60%, 70%, 90% or 95% of the level of binding activity of hemorrhagic fever virus specific antibody before treatment with the pathogen inactivation compound. In some embodiments of the invention, the level of binding activity of hemorrhagic fever virus specific antibody in the plasma after treatment with the pathogen inactivation compound is at least 80% of the level of binding activity of hemorrhagic fever virus specific antibody before treatment with the pathogen inactivation compound. In some embodiments of the methods, the level of binding activity of hemorrhagic fever virus specific antibody in the plasma after treatment with the pathogen inactivation compound is at least 50%, 60%, 70%, 90% or 95% of the level of binding activity of hemorrhagic fever virus specific antibody before treatment with the pathogen inactivation compound. In some embodiments of the invention, the method further comprises administering an antiviral agent.

The present disclosure further provides a method of treating a subject suffering from a hemorrhagic fever virus infection (*e.g.,* viral hemorrhagic fever), comprising administering to the subject a therapeutically effective amount of a plasma (*e.g.,* plasma preparation, plasma therapeutic) obtained from one or more plasma donors previously infected with or immunized against the same type (*e.g.,* same genus, same species, same subtype) of hemorrhagic fever virus (*e.g.,* same type of hemorrhagic fever virus as the subject), wherein the plasma is treated with a pathogen inactivation compound to inactivate pathogens, if present, and wherein the method reduces the level of hemorrhagic fever virus in the subject and treats (*e.g.,* reduces, decreases, suppresses, ameliorates) a condition associated with the hemorrhagic fever virus infection in the subject. In some instances, the plasma obtained from one or more plasma donors previously infected with or immunized against the same type of hemorrhagic fever virus is a plasma obtained from one or more plasma donors previously infected with the same type of hemorrhagic fever virus. In some instances, the condition associated with the hemorrhagic fever virus infection is coagulopathy. In some instances, the condition associated with the hemorrhagic fever virus infection is endothelial cell dysfunction. In some instances, the method treats two more conditions associated with the hemorrhagic fever virus infection in the subject. In some instances, the two or more conditions are coagulopathy and endothelial cell dysfunction. In some instances, the hemorrhagic fever virus is a bunyavirus, arenavirus, flavivirus or filovirus. In some instances, the hemorrhagic fever virus is a filovirus. In some instances, the filovirus is an Ebola virus (*e.g.,* Zaire ebolavirus, Bundibugyo ebolavirus, Reston ebolavirus, Sudan ebolavirus, Taï Forest ebolavirus) or a Marburg virus. In some instances, the hemorrhagic fever virus is an arenavirus. In some instances, the arenavirus is a Lassa fever virus, a Lujo virus, a Argentine hemorrhagic fever virus (*e.g.,* Junin virus), a Bolivian hemorrhagic fever virus (*e.g.,* Machupo virus), a Brazilian hemorrhagic fever virus (*e.g.,* Sabia virus), a Venezuelan hemorrhagic fever virus (*e.g.,* Guanarito virus) or Chapare hemorrhagic fever virus. In some instances, the hemorrhagic fever virus is a bunyavirus. In some instances, the bunyavirus is a Rift Valley fever virus, Crimean-Congo hemorrhagic fever virus, a Garissa virus, a Ilesha virus or a hantavirus (*e.g.,* Hantaan virus, Dobrava virus, Saaremaa virus, Seoul virus, Puumala virus). In some instances, the hemorrhagic fever virus is a flavivirus. In some instances, the flavivirus is a dengue fever virus, a yellow fever virus, Alkhurma homorrhagic fever virus, or a tick-borne encephalitis (*e.g.,* Omsk hemorrhagic fever virus, Kyasanur Forest disease virus). In some instances, the hemorrhagic fever virus is a rhabdovirus (*e.g.,* Bas-Congo virus, BASV).

In some instances of the aforementioned methods, the subject is a human subject. In some instances of the aforementioned methods, the plasma is obtained from one donor. In some instances, the plasma is of the same ABO blood group as the subject. In some instances of the aforementioned methods, the plasma is obtained from at least two donors. In some instances, the plasma is obtained from 2-12 donors. In some instances, the plasma is obtained from 2 donors, 3 donors, 4 donors, 5 donors, 6 donors, 7 donors, 8 donors, 9 donors, 10 donors, 11 donors or 12 donors. In some instances, the plasma comprises plasma obtained from donors only of the same ABO blood group as the subject. In some instances, the plasma comprises plasma obtained from donors of more than one ABO blood group. In some instances, the plasma comprises plasma obtained from donors of blood group A and blood group B. In some instances, the plasma is obtained from at least 3 donors and wherein the plasma comprises plasma obtained from donors of blood group A, blood group B and blood group AB. In some instances, the plasma does not contain plasma obtained from donors of blood group O.

In some instances of the aforementioned methods, the plasma obtained from one or more donors previously infected with or immunized against the hemorrhagic fever virus is obtained only from donors with no clinical symptoms of infection (*e.g.,* infection with the hemorrhagic fever virus) with the virus at the time of donating plasma. In some instances of the aforementioned methods, the plasma obtained from one or more donors previously infected with or immunized against the hemorrhagic fever virus is obtained only from donors that have no detectable levels of the virus in their (*i.e.,* the donor's) blood at the time of donating plasma. In some instances, no detectable levels of the virus in their blood is no detectable levels of the virus as determined by nucleic acid testing. In some instances, the virus is below the limit of detection in the donor's blood by nucleic acid testing. In some instances, nucleic acid testing is by PCR (*e.g.,* RT-PCR).

In some instances of the aforementioned methods, the plasma is frozen for storage and thawed prior to administration of the plasma to the subject. In some instances of the aforementioned methods, the plasma is lyophilized for storage and reconstituted prior to administration of the plasma to the subject. In some instances of the aforementioned methods, the plasma is a concentrated (*e.g.,* purified) immunoglobulin preparation. In some instances of the aforementioned methods, the plasma is obtained from the one or more donors by apheresis. In some instances, treatment with a pathogen inactivation compound is a photochemical treatment. In some instances of the aforementioned methods, the plasma is treated with a photoactive pathogen inactivation compound selected from the group consisting of a psoralen, an isoalloxazine, an alloxazine, a phthalocyanine, a phenothiazine, a porphyrin, and merocyanine 540. In some instances, the pathogen inactivation compound is a psoralen. In some instances, the pathogen inactivation compound is amotosalen.

In some instances of the aforementioned methods, the plasma obtained from one or more donors previously infected with or immunized against the hemorrhagic fever virus is a first donor plasma and the method further comprises administering a second donor plasma obtained from one or more donors not previously infected with or immunized against the same type of hemorrhagic fever virus (*i.e.,* same type of hemorrhagic fever virus as the subject), and wherein the second donor plasma is treated with a pathogen inactivation compound to inactivate pathogens, if present. In some instances, treatment with a pathogen inactivation compound is a photochemical treatment. In some instance, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some instances, the pathogen inactivation compound is a psoralen. In some instances, the pathogen inactivation compound is amotosalen.

In some instances of the aforementioned methods, the plasma obtained from one or more donors previously infected with or immunized against the hemorrhagic fever virus is a first donor plasma and the plasma further comprises a second donor plasma obtained from one or more donors not previously infected with or immunized against the same type of hemorrhagic fever virus (*i.e.,* same type of hemorrhagic fever virus as the subject), and wherein the second donor plasma is optionally treated with a pathogen inactivation compound to inactivate pathogens, if present. In some instances, treatment with a pathogen inactivation compound is a photochemical treatment. In some instances, the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540. In some instances, the pathogen inactivation compound is a psoralen. In some instances, the pathogen inactivation compound is amotosalen.

In some instances of the aforementioned methods, the second donor plasma is obtained from one donor. In some instances, the second donor plasma is of the same ABO blood group as the subject. In some instances of the aforementioned methods, the second donor plasma is obtained from at least two donors. In some instances, the second donor plasma is obtained from 2-12 donors. In some instances, the second donor plasma is obtained from 2 donors, 3 donors, 4 donors, 5 donors, 6 donors, 7 donors, 8 donors, 9 donors, 10 donors, 11 donors or 12 donors. In some instances, the second donor plasma comprises plasma only of the same ABO blood group as the subject. In some instances, the second donor plasma comprises plasma of more than one ABO blood group. In some instances, the second donor plasma comprises plasma of blood group A and blood group B. In some instances, the second donor plasma is obtained from at least 3 donors and wherein the second donor plasma comprises plasma of blood group A, blood group B and blood group AB. In some instances, the second donor plasma does not contain plasma of blood group O.

In some instances of the aforementioned methods, the second donor plasma is frozen for storage and thawed prior to administration of the plasma (*e.g.,* the second donor plasma) to the subject. In some instances of the aforementioned methods, the second donor plasma is lyophilized for storage and reconstituted prior to administration of the plasma (*e.g.,* the second donor plasma) to the subject. In some instances of the aforementioned methods, the second donor plasma is a plasma cryoprecipitate. In some instances of the aforementioned methods, the plasma (*e.g.,* plasma administered) comprises about 10-90% of the total volume as the first donor plasma and the remainder of the total volume as the second donor plasma. In some instances, the plasma (*e.g.,* plasma administered) comprises about 10-50% of the total volume as the first donor and the remainder of the total volume as the second donor plasma. In some instances, the plasma (*e.g.,* plasma administered) comprises about 10-80%, about 10-70%, about 10-60%, about 10-40%, about 10-30% or about 10-20% of the total volume as the first donor plasma and the remainder of the total volume as the second donor plasma. In some instances, the plasma (*e.g.,* plasma administered) comprises about 10%, about 20%, about 30%, about 40%, about 50%, about 60%, about 70%, about 80% or about 90% of the total volume as the first donor plasma and the remainder of the total volume as the second donor plasma. In some instances, the plasma (*e.g.,* plasma administered) comprises a volume ratio of about 1:1 for the second donor plasma and the first donor plasma. In some instances, the plasma (*e.g.,* plasma administered) comprises a volume ratio of about 2:1 for the second donor plasma and the first donor plasma. In some instances, the plasma (*e.g.,* plasma administered) comprises a volume ratio of at least about 3:1, at least about 4:1, at least about 5:1, at least about 6:1, at least about 7:1, at least about 8:1, at least about 9:1 or at least about 10:1 for the second donor plasma and the first donor plasma. In some instances, the plasma (*e.g.,* plasma administered) comprises less than about 50% of the total volume as the first donor plasma.

In some instances of the aforementioned methods, the first donor plasma and the second donor plasma are administered separately to the subject. In some instances, the first donor plasma and the second donor plasma administered sequentially to the subject. In some instances, the first donor plasma and the second donor plasma are administered within about 72 hours, within about 48 hours, within about 24 hours, within about 15 hours, within about 8 hours, within about 4 hours, within about 2 hours, or within about 1 hour of each other. In some instances, the second donor plasma is administered before the first donor plasma. In some instances, the second donor plasma is administered as a plasma exchange (*e.g.,* plasmapheresis, therapeutic plasma exchange). In some instances, the plasma exchange comprises about 1.0 to 1.5 blood volumes of plasma (*e.g.,* total plasma volume of the subject). In some instances, the plasma exchange comprises a total (*e.g.,* 100%) plasma volume. In some instances, the plasma exchange volume is adjusted (*e.g.,* increased) relative to a dilution of the plasma from a pathogen inactivation process (*e.g.,* treated with a pathogen inactivation compound). In some instances, the first donor plasma and the second donor plasma are administered to the subject at about the same time. In some instances, the first donor plasma and the second donor plasma are mixed prior to or during administration to the subject.

In some instances of the aforementioned methods, the plasma is administered to the subject in a volume (*e.g.,* total volume, total volume of one or more units) of about 200-2500 mL, about 200-2000mL, about 200-1500 mL, about 200-1000 mL or about 200-500 mL. In some instances, the plasma is administered to the subject in a volume of about 300-2500 mL. In some instances, the plasma is administered to the subject in a volume of about 300-2000 mL. In some instances, the plasma is administered to the subject in a volume of about 300-1500 mL. In some instances, the plasma is administered to the subject in a volume of about 300-1200 mL, about 300-1000 mL, about 300-800 mL, about 300-700 mL, about 300-600 mL, about 300-500 mL or about 300-400 mL. In some instances, the plasma is administered to the subject in a volume of about 400-500 mL or 400-600 mL. In some instances, the plasma is administered to the subject in volume of about 15-20 mL/kg body weight of the subject (*e.g.,* weight adjusted dosing). In some instances, the plasma is administered to the subject in volume of about 40-60 mL/kg body weight of the subject. In some instances, the plasma is administered to the subject in volume of about 10 mL/kg, about 15 mL/kg, about 20 mL/kg, about 25 mL/kg, about 30 mL/kg, about 40 mL/kg, about 50 mL/kg or about 60 mL/kg body weight of the subject. In some instances, the plasma is administered by infusion. In some instances, the plasma is administered to the subject as one or more infusions, two or more infusions, three or more infusions or four or more infusions.

In some instances of the aforementioned methods, the level of viral infection (*e.g.,* viral load, virus titer) in the subject is reduced. In some instances, the level of viral infection is reduced to no detectable hemorrhagic fever virus in blood of the subject. In some instances, no detectable hemorrhagic fever virus in blood of the subject is no detectable hemorrhagic fever virus as determined by nucleic acid testing. In some instances, the viral infection is reduced in the subject's blood below the limit of detection by nucleic acid testing. In some instances, nucleic acid testing is by PCR (*e.g.,* RT-PCR). In some instances, the mortality associated with the virus infection is reduced. In some instances, the number of co-morbidities or severity of morbidity (*e.g.,* one or more morbidities) is reduced (*e.g.,* decreased, ameliorated). In some instances, the absence of clinical symptoms indicative of viral hemorrhagic fever virus disease is achieved. In some instances, coagulopathy is reduced. In some instances, coagulopathy (*e.g.,* the degree of coagulopathy) is determined by one or more analytical measures known in the art for assessing coagulopathy (see *e.g.,* Bates et al., 2005, Circulation 112:e53-e60), such as for example, thrombin generation, prothrombin time (PT), international normalized ratio (INR), activated partial thromboplastin time (aPTT), fibrinogen and platelet count. In some embodiments, endothelial cell dysfunction (*e.g.,* damage) is decreased. In some instances, endothelial cell function (*e.g.,* associated with damage from the virus infection) is restored (*e.g.,* partially restored). Endothelial cell dysfunction/function may be determined by any of a variety of measures known in the art (see *e.g.,* Meigs et al., 2004, JAMA 291:1978-1986; Deanfield et al., 2007, Circulation 115:1285-1295; Xing et al., 2012, Critical Care 16:R7; Shapiro et al., 2010, Critical Care 14:R182). In some embodiments, endothelial cell function or dysfunction is determined by measurement of one or more biomarkers selected from the group consisting of von Willebrand factor (vWF), ADAMTS13, angiopoietins (Ang)-1 and -2, endocan, selectins (*e.g.,* E-, P-, L-), endothelial leukocyte adhesion molecule (E-selectin or ELAM-1), endothelin (ET-1), endothelin precursor peptide proET-1, VEGF, soluble VEGF-receptor-1 (Flt-1), PDGF, plasminogen activator inhibitor (PAI-1), urokinase PA (uPA) and fibrin degradation products (*e.g.,* X and Y fragments, D-dimers, D and E fragments, Bβ15-42). In some instances, the endothelial barrier function is enhanced. In some instances, time of hospitalization is reduced. In some instances, time spent in ICU is reduced. In some instances, total duration or frequency of dialysis is decreased. In some instances, time on assisted ventilation is decreased. In some instances, incidence of organ failure is reduced. In some instances, multifocal necrosis (e.g., multifocal hepatic necrosis) associated with virus infection is reduced.

In some instances of the aforementioned methods, the level of binding activity of hemorrhagic fever virus specific antibody in the plasma obtained from one or more donors previously infected with or immunized against the hemorrhagic fever virus, after treatment with the pathogen inactivation compound, is at least 80% of the level of binding activity of hemorrhagic fever virus specific antibody before treatment with the pathogen inactivation compound. In some instances of the aforementioned methods, the level of binding activity of hemorrhagic fever virus specific antibody in the donor plasma obtained from one or more donors previously infected with or immunized against the hemorrhagic fever virus, after treatment with the pathogen inactivation compound, is at least 50%, 60%, 70%, 90% or 95% of the level of binding activity of hemorrhagic fever virus specific antibody before treatment with the pathogen inactivation compound. In some instances of the aforementioned methods, the level of binding activity of hemorrhagic fever virus specific antibody in the plasma after treatment with the pathogen inactivation compound is at least 80% of the level of binding activity of hemorrhagic fever virus specific antibody before treatment with the pathogen inactivation compound. In some instances of the aforementioned methods, the level of binding activity of hemorrhagic fever virus specific antibody in the plasma after treatment with the pathogen inactivation compound is at least 50%, 60%, 70%, 90% or 95% of the level of binding activity of hemorrhagic fever virus specific antibody before treatment with the pathogen inactivation compound. In some instances of the aforementioned methods, the method further comprises administering an antiviral agent.

It is to be understood that one, some, or all of the properties of the various embodiments described herein may be combined to form other embodiments. These and other aspects will become apparent to one of skill in the art. These and other embodiments are further described by the detailed description that follows.

### DETAILED DESCRIPTION

### Definitions

The terms "treating", "treat" and "treatment" as used with respect to the methods described herein refer to eliminating, reducing, suppressing or ameliorating, either temporarily or permanently, either partially or completely, a clinical symptom, manifestation or progression of an event, disease or condition, such as, for example, hemorrhagic fever virus infections and conditions associated with such infections. Such treating need not be absolute to be useful.

The terms "effective amount", "therapeutically effective amount" and "amount effective to treat" as used herein refers to at least an amount effective, at dosages and for periods of time necessary, to achieve a desired therapeutic result, such as for example, a detectable (*e.g.,* measureable), positive effect or improvement on any symptom, aspect, parameter or characteristics of a disease state or condition when administered to a subject. An effective amount can be provided in one or more administrations. Such effect amount need not be absolute to be beneficial.

The term "amotosalen" means the compound 3-(2-aminoethoxymethyl)-2,5,9-trimethylfuro[3,2-g]chromen-7-one and any salts thereof. The compound may also be referred to as 3-[(2-aminoethoxy)methyl]-2,5,9-trimethyl-7H-furo[3,2-G][1]benzopyran-7-one-hydrochloride. The compound may also be referred to as 4'-(4-amino-2-oxa)butyl-4,5',8-trimethyl psoralen. Where the inactivation of plasma includes adding amotosalen HCl (the HCl salt of amotosalen) to plasma, such as a unit of plasma (*e.g.,* donor plasma), the removal of this compound from the plasma is not limited to the removal of amotosalen HCl, as the amotosalen can be present in solution as other salts or as the free base. As used in the methods described herein, removal of amotosalen means removal of the compound in any form, *e.g.* as the free base or as any salt, as measured by the assays described herein. Treatment or processing of plasma by amotosalen inactivation refers to combining plasma (*e.g.,* unit of plasma, individual unit, pooled units) with amotosalen and illuminating with a suitable dose of UVA light in order to inactivate pathogens that may be present in the plasma. In some embodiments, amotosalen inactivated plasma have been pathogen inactivated according to commercial methods for plasma, or by similar methods. Such methods provide, for example, a plasma unit prior to addition of amotosalen having a volume within the range of 385 to 650 mL, which is combined with either 15 mL of a 3 mM amotosalen solution, resulting in a plasma unit ready for illumination having an amotosalen concentration within the range of about 120 to 193 µM (nominal concentration 150 µM) prior to illumination. The resulting solution is illuminated with the equivalent of about 3 J/cm² of light in the UVA wavelength range. While the methods described herein are applied to plasma units using known systems such as the use of amotosalen for pathogen inactivation in plasma, they are applicable to any pathogen inactivated unit of plasma.

### Plasma

Plasma is the largest component of blood, making up about 55 percent of its overall content, and when isolated, is a light yellow, straw-colored liquid. Along with water, salts and enzymes, plasma also contains immunoglobulins (antibodies), clotting factors, and the proteins albumin and fibrinogen. The components of the plasma are well known in the art (see *e.g.,* Philip Westerman, Plasma Proteins, VII-1 to VIII-13, Sep. 17, 2002). Serum is also well defined and generally called as blood plasma without fibrinogen and other clotting factors. The source of plasma used in the composition of the present disclosure is preferably of the same mammalian species as the subject (*e.g.,* human).

Plasma for use in the compositions and methods described herein can be readily obtained (*e.g.,* collected) using conventional methods well known in the art. Plasma can be obtained from donated whole blood using standard methods (*e.g.,* processing methods, separation methods) such as centrifugation, sedimentation and filtration, sufficient to remove desired amounts of platelets, red blood cells and white blood cells. Plasma can also be obtained by apheresis, which involves collecting plasma using a cell separator or apheresis machine and returning uncollected cells (*e.g.,* red blood cells, platelets) to the donor at the time of donation (see *e.g.,* COBE® Spectra Apheresis System, Trima Accel® Automated Blood Collection System, Terumo BCT), allowing larger and more frequent donations.

Plasma can be stored in various forms prior to use, including, for example, frozen plasma (*e.g.,* fresh-frozen, FP-24) preparation, cryoprecipitated plasma preparation (*e.g.,* plasma cryoprecipitate), lyophilized plasma preparation (*e.g.,* freeze-dryed, spray-dried) or concentrated plasma (*e.g.,* concentrated Ig) preparation. Fresh-frozen plasma may be frozen at a temperature of less than about -18° C (*e.g.,* about -18° C to -40° C), following centrifugation within the particular time allotted for the collection system (*e.g.,* six hours, within eight hours) from blood collection, while the FP-24 may be similarly frozen within 24 hours of collection. Lyophilized plasma may be prepared using know techniques (see *e.g.,* Hellstern et al., 1992, Vox Sang. 63:178-185). Cryoprecipitated plasma may be obtained, for example, by freezing (*e.g.,* dry ice/ethanol bath) and then thawing frozen (*e.g.,* fresh-frozen) plasma in a refrigerator or controlled water bath (*e.g.,* at the temperature of 4° C) until "slushy", to form white precipitate (cold precipitated protein), isolating the formed precipitate by centrifugation and separation from excess plasma, and refreezing at the temperature of from about -18° C to -40° C for storage. For example, concentrated plasma preparations may be obtained for example, by mixing with a thickener such as dextranomer, SEPHADEX, dextramine, polyacrylamide, BIO-GEL P, silica gel, zeolite, DEBRISAN, crosslinked agarose, starch and alginate gel and discarding the remaining thickener, by fractionation or by other purification methods (*e.g.,* concentrated plasma immunoglobulin preparation may be obtained by standard purification methodologies, such as for example, using anion-exchange chromatography (see *e.g.,* U.S. Patent 6,093,324)).

Plasma may be obtained from one or more donors not previously infected with or immunized against a hemorrhagic fever virus, such as for example, the same type of hemorrhagic fever virus as a subject suffering from a hemorrhagic fever virus infection (*e.g.,* recipient of such plasma). Alternatively, or in addition, plasma may be obtained from one or more donors previously infected with or immunized against a hemorrhagic fever virus, such as for example, the same type of hemorrhagic fever virus as a subject suffering from a hemorrhagic fever virus infection (*e.g.,* recipient of such plasma). In certain embodiments, plasma obtained from one or more donors previously infected with or immunized against a hemorrhagic fever virus is plasma obtained from one or more donors previously infected with a hemorrhagic fever virus. Such donors previously infected with or immunized against a hemorrhagic fever virus may have no clinical symptoms of infection with the hemorrhagic fever virus and/or no detectable levels of a hemorrhagic fever virus in their blood at the time of donating the plasma as described herein. Donors may be tested to determine the presence or absence of detectable levels (*e.g.,* below the limit of detection) of a hemorrhagic fever virus in their blood (*e.g.,* whole blood, plasma) using any method known in the art. For example, the presence or absence of detectable hemorrhagic fever virus may be determined by any number of accepted molecular and/or virological techniques known in the art, such as for example, nucleic acid testing and/or cell based infectivity assays. Nucleic acid testing methodologies (*e.g.,* RT-PCR, qPCR) are known in the art, including for use in testing blood products for transfusion, and may be applied to any virus for which a nucleic acid sequence (*e.g.,* genome sequence, partial nucleotide sequence) has been determined. A variety of cell based infectivity assays are also known and may include, for example, plaque assay, TCID₅₀ assays, in situ staining assays, hemagglutination assay, anucleic acid testing of infected cells (*e.g.,* RT-PCR, qPCR), ELISA assay and flow cytometric assay.

### Pathogen Inactivation

Blood products, including plasma preparations, may contain pathogens, or may be contaminated with pathogens during processing. As such, it is desirable to subject such blood products to a process in order to reduce the risk of transfusion-transmitted diseases. Various methods have been assessed to mitigate the risk of transfusion-associated disease transmission. Aside from screening and detection of pathogens and subsequent elimination of contaminated blood products, processes that incorporate treatments to inactivate pathogens (*i.e.,* pathogen inactivation) that may be present are available. Ideally, such a process results in the inactivation (*e.g.,* greater than 1 log, greater than 2 logs, greater than 3 logs, greater than 4 logs, greater than 5 logs, greater than 6 logs or more inactivation) of a broad range of pathogens such as viruses, bacteria and parasites that may be present in the blood product. In certain embodiments, the methods of pathogen inactivation utilize a solvent/detergent treatment process, such as processes known in the art. In certain preferred embodiments, the methods of pathogen inactivation require addition of an amount of pathogen inactivation compound to a unit of plasma. For example, pathogen inactivation may involve the addition of a low molecular weight compound that inactivates various pathogens, such as for example, the addition of a photosensitizer that, when activated by illumination using light of suitable wavelengths, will inactivate a variety of pathogens that may be present. Two exemplary methods currently developed include the addition of amotosalen (INTERCEPT™ Blood System, Cerus Corporation) or riboflavin (MIRASOL™, Terumo BCT) to the plasma, with subsequent illumination with UV light. Other methods include, for example, illumination with UV light without addition of a photosensitizer, as well as illumination with other photoactive compounds, including psoralen derivatives other than amotosalen, isoalloxazines other than riboflavin, alloxazines, dyes such as phthalocyanines, phenothiazine dyes (*e.g.* methylene blue, azure B, azure C, thionine, toluidine blue), porphyrin derivatives (*e.g.* dihematoporphyrin ether, hematoporphyrin derivatives, benzoporphyrin derivatives, alkylsubstituted sapphyrin), and merocyanine 540 (Prodouz et al., Blood Cells 1992, 18(1):101-14; Sofer, Gail, BioPharm, August 2002). Other pathogen inactivation systems include, for example, those described in PCT publication numbers WO 2012071135; WO 2012018484; WO 2003090794; WO 2003049784; WO 1998018908; WO 1998030327; WO 1996008965; WO 1996039815; WO 1996039820; WO 1996040857; WO 1993000005; US patent application number US 20050202395; and US patent numbers 8296071 and 6548242, the disclosures of which are hereby incorporated by reference as they relate to pathogen inactivation in blood products. Where addition of a compound to the plasma is used for pathogen inactivation, whether the method requires illumination or not, in some instances it is desirable to remove any residual pathogen inactivation compound or by-product thereof. Methods for pathogen inactivation and removal of pathogen inactivation compound as described herein are applicable to any plasma units, whether prepared from whole blood donation or apheresis.

It is understood that a solution of pathogen inactivation compound can be added during the processing to inactivate pathogens, since pathogen inactivating compound is not typically combined in solid form, but is dissolved in a solution (for example, amotosalen is the HCl salt dissolved in a saline solution). As such, when a unit of plasma is treated for pathogen inactivation, some volume of the solution of pathogen inactivating compound will be included in the final product, as well as some volume of any anticoagulant used in collecting the blood for isolation of plasma. For administration of plasma to a subject as disclosed herein, the volume of plasma administered (*e.g.,* exchange volume) may be adjusted (*e.g.,* increased) relative to a dilution of the plasma from a pathogen inactivation process (*e.g.,* addition of pathogen inactivation compound) and/or an added anticoagulant.

Some pathogen inactivation methods may require the use of a removal device, *i.e.* a device for reducing the concentration of pathogen inactivation compound, such as a small organic compound, and by-products thereof in a unit of plasma while substantially maintaining a desired biological activity of the plasma. Such a removal device is preferably intended to be used in a flow through mode, *i.e.* the plasma is passed through the removal device, following illumination, and prior to collection in a storage bag or other container. Such devices entail the use of an adsorbent particle that binds the pathogen inactivation compound. Adsorbent particles may be immobilized within a matrix, within the removal device.

In some instances, the removal device comprises porous adsorbent particles in an amount sufficient to reduce the pathogen inactivation compound to below a desired concentration, wherein the adsorbent particles have an affinity for the pathogen inactivation compound, where it is understood such adsorbent particles can be selected to best adsorb the compound or compounds to be removed, with minimal effect on components that should not be removed or damaged by contact with the adsorbent particles. A variety of adsorbent particles are known, including generally particles made from any natural or synthetic material capable of interacting with compounds to be removed, including particulates made of natural materials such as activated carbon, silica, diatomaceous earth, and cellulose, and synthetic materials such as hydrophobic resins, hydrophilic resins or ion exchange resins. Such synthetic resins include, for example, carbonaceous materials, polystyrene, polyacrylic, polyacrylic ester, cation exchange resin, and polystyrene-divinylbenzene. Detailed description of such removal devices suitable for use in the methods as described herein can be found, for example, in PCT publication numbers WO 1996040857, WO 1998030327, WO 1999034914, and WO 2003078023,. Exemplary adsorbent particles include, but are not limited to, Amberlite (Rohm and Haas) XAD-2, XAD-4, XAD-7, XAD-16, XAD-18, XAD-1180, XAD-1600, XAD-2000, XAD-2010; Amberchrom (Toso Haas) CG-71m, CG-71c, CG-161m, CG161c; Diaion Sepabeads (Mitsubishi Chemicals) HP20, SP206, SP207, SP850, HP2MG, HP20SS, SP20MS; Dowex (Dow Chemical) XUS-40285, XUS-40323, XUS-43493 (also referred to as Optipore V493 (dry form) or Optipore L493 (hydrated form)), Optipore V503, Optipore SD-2; Hypersol Macronet (Purolite) MN-100, MN-102, MN-150, MN-152, MN-170, MN-200, MN-202, MN-250, MN-252, MN-270, MN-300, MN-400, MN-500, MN-502, Purosorb (Purolite) PAD 350, PAD 400, PAD 428, PAD 500, PAD 550, PAD 600, PAD 700, PAD 900, and PAD 950. Material used to form an immobilized matrix may comprises a low melting polymer, such as nylon, polyester, polyethylene, polyamide, polyolefin, polyvinyl alcohol, ethylene vinyl acetate, or polysulfone. In one example, the adsorbent particles immobilized in a non-porous thermoplastic binder. While it is understood that the methods and devices described herein encompass removal devices as are known in the art, such methods and devices may be exemplified using the removal device of an amotosalen inactivated plasma product as is commercially available (CAD, INTERCEPT™ Blood System, Cerus Corp). Such a removal device comprises Hypersol Macronet MN-250 adsorbent contained with a binder, such as a UHMW-PE binder.

### Treatment methods

The compositions and their use provided herein relate to treatment of subjects suffering from a hemorrhagic fever virus infection, including for example, treating viral hemorrhagic fever and associated conditions with one or more plasma preparations. Representative hemorrhagic fever viruses contemplated by the present disclosure include, for example, filoviruses (*e.g.,* Ebola virus, Marburg virus), arenaviruses (*e.g.,* Lassa virus, Junin virus), bunyaviruses (*e.g.,* Rift Valley fever virus, Crimean-Congo hemorrhagic fever virus), flaviviruses (*e.g.,* dengue fever virus, Omsk hemorrhagic fever virus), and certain rhabdovirues.

The disclosure provides, for example, methods of treating a subject suffering from a hemorrhagic fever virus infection, comprising administering to the subject a therapeutically effective amount of a plasma (*e.g.,* plasma preparation, plasma therapeutic), wherein the plasma comprises a first plasma component obtained from one or more plasma donors previously infected with or immunized against the same type (*e.g.,* same genus, same species, same subtype) of hemorrhagic fever virus (*i.e.,* same type of hemorrhagic fever virus as the subject) and a second plasma component obtained from one or more plasma donors not previously infected with or immunized against the same type of hemorrhagic fever virus (*i.e.,* same type of hemorrhagic fever virus as the subject).

Also disclosed are methods of treating a condition associated with a hemorrhagic fever virus infection in a subject, comprising administering a plasma (*e.g.,* plasma preparation, plasma therapeutic) in an amount effective to treat (*e.g.,* reduce, decrease, prevent, suppress, ameliorate) the condition, wherein the plasma comprises donor plasma obtained from one or more donors previously infected with or immunized against the same type (*e.g.,* same genus, same species, same subtype) of hemorrhagic fever virus (*i.e.,* same type of hemorrhagic fever virus as the subject), and wherein the plasma or donor plasma is treated with a pathogen inactivation compound to inactivate pathogens, if present. Such methods may further comprise plasma (*e.g.,* a second donor plasma) obtained from one or more donors not previously infected with or immunized against the same type of hemorrhagic fever virus (*i.e.,* same type of hemorrhagic fever virus as the subject). For example, in certain embodiments, the condition is an active (*e.g.,* acute) viral infection (*e.g.,* viral load, infectious virus), and the method comprises a method of treating a condition associated with a hemorrhagic fever virus infection in a subject, comprising administering a plasma (*e.g.,* plasma preparation, plasma therapeutic) in an amount effective to treat (*e.g.,* reduce, decrease, prevent, suppress, ameliorate) the active viral infection, wherein the plasma comprises donor plasma obtained from one or more donors previously infected with or immunized against the same type (*e.g.,* same genus, same species, same subtype) of hemorrhagic fever virus, and wherein the plasma or donor plasma is treated with a pathogen inactivation compound to inactivate pathogens, if present. Alternatively or in addition, in certain embodiments, the condition is coagulopathy or endothelial cell dysfunction (or both), and the method comprises a method of treating a condition associated with a hemorrhagic fever virus infection in a subject, comprising administering a plasma (*e.g.,* plasma preparation, plasma therapeutic) in an amount effective to treat (*e.g.,* reduce, decrease, prevent, suppress, ameliorate) coagulopathy or endothelial cell dysfunction, wherein the plasma comprises donor plasma obtained from one or more donors previously infected with or immunized against the same type (*e.g.,* same genus, same species, same subtype) of hemorrhagic fever virus, and wherein the plasma or donor plasma is treated with a pathogen inactivation compound to inactivate pathogens, if present. In certain instances of the methods disclosed herein, plasma is administered in an amount effective to ameliorate the condition (*i.e.,* to improve, lessen the severity of, alleviate and/or reduce the intensity and/or number of symptoms associated with the condition).

The present disclosure also provides methods of treating a subject suffering from a hemorrhagic fever virus infection (*e.g.,* viral hemorrhagic fever), comprising administering to the subject a therapeutically effective amount of a plasma (*e.g.,* plasma preparation, plasma therapeutic) obtained from one or more plasma donors previously infected with or immunized against the same type (*e.g.,* same genus, same species, same subtype) of hemorrhagic fever virus (*e.g.,* same type of hemorrhagic fever virus as the subject), wherein the plasma is treated with a pathogen inactivation compound to inactivate pathogens, if present, and wherein the method reduces the level of hemorrhagic fever virus in the subject and treats (*e.g.,* reduces, decreases, suppresses, ameliorates) at least one condition associated with the hemorrhagic fever virus infection in the subject. Such methods may further comprise plasma (*e.g.,* a second donor plasma) obtained from one or more donors not previously infected with or immunized against the same type of hemorrhagic fever virus (*i.e.,* same type of hemorrhagic fever virus as the subject). The condition associated with the hemorrhagic fever virus infection may be for example, coagulopathy and/or endothelial cell dysfunction. The method may also treat two more, three or more, four or more five or more conditions associated with the hemorrhagic fever virus infection in the subject. It should be understood that in this aspect of the disclosure, treatment of a condition (e.g., one or more conditions) associated with the hemorrhagic fever virus infection in the subject refers to condition(s) in addition to reducing the level of hemorrhagic fever virus in the subject, and as such both a reduction in the level of hemorrhagic fever virus and treatment of one or more conditions as provided herein are contemplated. Exemplary conditions associated with the hemorrhagic fever virus infection that may be treated as disclosed herein include coagulopathy and/or endothelial cell dysfunction.

The aforementioned compositions and methods of the present disclosure provide plasma comprising, for example, plasma (*e.g.,* donor plasma) obtained from one or more donors (*e.g.,* plasma donors) previously infected with or immunized against the same type of hemorrhagic fever virus as the subject to which administration of such plasma is intended. The disclosure contemplates the same type of hemorrhagic fever virus to mean, for example, a hemorrhagic fever virus of the same genus, and in certain instances the same species, and in certain embodiments the same subtype. Generally plasma obtained from a donor previously infected with or immunized against the same type of hemorrhagic fever virus will be from a donor previously infected with or immunized against a hemorrhagic fever virus sufficiently similar (*e.g.,* immunogenically similar, immunogenically related) to elicit cross-reactive immune response(s), such as for example cross-reactive antibody (*e.g.,* neutralizing antibody) and/or other antigen-specific elements of the immune system.

Infusion methods for plasma are well known in the art of transfusion medicine and may be readily applied as a preferred method for administering the plasma preparations, as disclosed herein. In certain instances, plasma is preferably administered on a weight adjusted basis (*e.g.,* weight adjusted dosing), but may also be administered as a fixed volume dose independent of the subject's weight, each as described herein. For example, plasma may be administered to a subject in volume (*e.g.,* dosage) of about 10 mL/kg to about 60 mL/kg body weight of the subject (*e.g.,* about 15-20 mL/kg, about 40-60 mL/kg body, about 10 mL/kg, about 15 mL/kg, about 20 mL/kg, about 25 mL/kg, about 30 mL/kg, about 40 mL/kg, about 50 mL/kg, about 60 mL/kg). Alternatively, or in addition, plasma may be administered to a subject in a volume (*e.g.,* dosage, total volume, total volume of one or more units) of about 200-2500 mL, about 200-2000mL, about 200-1500 mL, about 200-1000 mL, about 200-500 mL, about 300-2500 mL, or about 300-2000 mL. In certain instances, plasma may be administered to a subject in a volume of about 300-1500 mL (*e.g.,* about 300-1200 mL, about 300-1000 mL, about 300-800 mL, about 300-700 mL, about 300-600 mL, about 300-500 mL, about 300-400 mL, about 400-500 mL, about 400-600 mL). In certain instances, the plasma may be administered to the subject as a single infusion. The methods herein also contemplate administering one or more, two or more, three or more, four or more or five or more infusions. For example, in certain instances, a 1000 mL volume of plasma (*e.g.,* from a single donor, from multiple donors) may be administered as two infusions of about 500 mL per infusion, or a 500 mL volume of plasma (*e.g.,* from a single donor, from multiple donors) may be administered as two infusions of about 250 mL per infusion, or a 300 mL volume of plasma (*e.g.,* from a single donor, from multiple donors) may be administered as two infusions of about 150 mL per infusion. Further, infusions as provided herein may comprise, for example, a plasma from a single container (*e.g.,* plasma bag) administered by a single intravenous transfusion line, including for example, single donor plasma or pooled (*e.g.,* mixed) donor plasma, as well as plasma from multiple containers (*e.g.,* obtained from a single donor, obtained from multiple donors) administered sequentially by the same or different transfusion lines, including for example, in certain instances, separate containers for first and second donor plasmas, or first and second plasma components. In certain instances, plasma from two or more plasma containers may flow into the same transfusion line for administration at about the same time by way of one or more connectors (e.g., branch connectors) enabling such administration (*e.g.,* co-administration, mixing).

When administered from multiple (*e.g.,* separate) containers, such as two or more plasma bags, administration of plasma from the two or more plasma bags (*e.g.,* a first donor plasma or first plasma component, and a second donor plasma or second plasma component) are generally within about 72 hours, about 48 hours, about 24 hours, within about 15 hours, within about 8 hours, within about 4 hours, within about 2 hours, within about 1 hour of each other, or in certain instances, immediately after each other or at about the same time. The disclosure contemplates any desired order of administration when plasma is provided from more than one container, such as, for example, a second donor plasma or second plasma component being administered before a first donor plasma or first plasma component. In some instances, the second donor plasma or second plasma component is administered as a plasma exchange (*e.g.,* therapeutic plasma exchange).

In some instances, the present disclosure provides a plasma comprising a first plasma component or first donor plasma, and a second plasma component or second donor plasma, and the plasma comprises about 10-90% of the total volume as the first plasma component or first donor plasma and the remainder of the total volume as the second plasma component or second donor plasma, respectively, such as for example, the plasma comprises about 10-50% of the total volume as the first plasma component or first donor plasma and the remainder of the total volume as the second plasma component or second donor plasma; the plasma comprises about 10-80%, about 10-70%, about 10-60%, about 10-40%, about 10-30% or about 10-20% of the total volume as the first plasma component or first donor plasma and the remainder of the total volume as the second plasma component or second donor plasma; the plasma comprises about 10%, about 20%, about 30%, about 40%, about 50%, about 60%, about 70%, about 80% or about 90% of the total volume as the first plasma component or first donor plasma and the remainder of the total volume as the second plasma component or second donor plasma. The disclosure also provides in some instances, that the plasma comprises a volume ratio of about 2:1 for the second plasma component and the first plasma component, or the second donor plasma and the first donor plasma; that the plasma comprises a volume ratio of at least about 3:1, at least about 4:1, at least about 5:1, at least about 6:1, at least about 7:1, at least about 8:1, at least about 9:1 or at least about 10:1 for the second plasma component and the first plasma component, or the second donor plasma and the first donor plasma; or that the plasma comprises less than about 50% of the total volume as the first plasma component or the first donor plasma.

Following treatment administration, measures of a treatment effect (*e.g.,* efficacy) may vary according to the particular hemorrhagic fever virus, severity and/or morbidities of disease and associated conditions. Generally, survival is a key indicator of efficacy in severe viral hemorrhagic fever. Reduction or elimination of active viral infection (*e.g.,* infectious virus) in the subject (*e.g.,* greater than 1 log, greater than 2 logs, greater than 3 logs, greater than 4 logs, greater than 5 logs, greater than 6 logs, greater than 7 logs, greater than 8 logs or more) provides another measurement of efficacy, and may be determined by any number of accepted molecular and/or virological techniques known in the art, such as for example, nucleic acid testing (*e.g.,* in blood) and cell based infectivity assays (see *e.g.,,* Barzon et al., 2013, J. Clin. Virol. 58:346-350; Sidoti et al., 2013, Mol. Biotechnol. 53:352-362; Sedlak et al., 2013, Diag. Micro. Inf. Dis. 75:1-4). Nucleic acid testing methodologies (*e.g.,* RT-PCR, qPCR) are known in the art, including blood products for transfusion, and may be applied to any virus for which a nucleic acid sequence (*e.g.,* genome sequence) has been determined. A variety of cell based infectivity assays are also known and may include, for example plaque assay, TCID₅₀ assays, in situ staining assays, hemagglutination assay, nucleic acid testing of infected cells (*e.g.,* RT-PCR, qPCR), ELISA assay, flow cytometric assay and the like. Reducing the level of viral infection to no detectable hemorrhagic fever virus in blood as provided in the present disclosure may, in certain embodiments, mean reducing the virus to below a detection limit of 100, below a detection limit of 50, below a detection limit of 20, or below a detection limit of 10 genome copies/mL of blood. Additional assessments may include, for example, subject hemostatic function (*e.g.* improvement), amelioration of one or more clinical symptoms, decrease in hemodialysis requirements, decrease in assisted ventilation requirements, decrease in time of hospitalization, decrease in time spent in ICU, enhanced endothelial barrier function, reduction in incidence of organ failure, reduction in multifocal necrosis associated with virus infection and presence or absence of markers of endothelial cell function or dysfunction, as described herein. Examples of established assay methods to determine treatment effect for one or more of the aforementioned, include for example, complete blood count (CBC), platelet count, international normalized ratio (INR), activated clotting time (ACT), prothrombin time (PT, Protime, Quick's time, Partial Prothrombin Time), activated partial thromboplastin time (aPTT, Activated Prothrombin Time), thrombin time (Thrombin Clotting Time, TCT, TT) and plasma fibrinogen level determination (*e.g.,* von Clauss technique). Such assays may be performed manually or using a variety of semi- or automated instruments, such as for example, microprocessor-controlled instruments, including those manufactured by Helena Laboratories Corp. (Beaumont, Texas), ITC (Edison, NJ), Medtronics (Minneapolis, MN), and Roche Diagnostics (Indianapolis, IN).

Coagulopathy (*e.g.,* the degree of coagulopathy) may be determined by one or more analytical measures known in the art (see e.g., Lance 2015 Thrombosis Journal 13:1-6; Bates et al., 2005, Circulation 112:e53-e60; Castoldi et al., 2011, Thromb. Res. 127 Suppl. 3:S21-25). For example, coagulopathy may be assessed using assays such as thrombin generation, prothrombin time (PT), international normalized ratio (INR), activated partial thromboplastin time (aPTT), fibrinogen and platelet count. A treatment effect on coagulopathy may be demonstrated for example, when coagulation factor levels are at least 10%, at least 20%, at least 30%, at least 40%, at least 50% or more of normal. Alternatively, or in addition, treatment effect may be demonstrated when the fibrinogen level is above 100mg/dL, above 110 mg/dL, above 120 mg/dL, above 130 mg/dL, above 140 mg/dL or above 150 mg/dL. Alternatively, or in addition, treatment effect may be demonstrated when APTT of less than about 50 sec, less than about 45 sec, less than about 35 sec or less than about 30 sec is achieved. Alternatively, or in addition, treatment effect may be demonstrated when PT of less than about 18 sec, less than about 17 sec, less than about 16 sec, less than about 15 sec. or less than about 14 sec is achieved.

Endothelial cell dysfunction and changes thereto (e.g., improvement, enhancement, decreased damage, partially or fully restored function) may be determined by any of a variety of methods and/or assays known in the art for assessing endothelial cell dysfunction/function (see *e.g.,* Meigs et al., 2004, JAMA 291:1978-1986; Deanfield et al., 2007, Circulation 115:1285-1295; Xing et al., 2012, Critical Care 16:R7; Shapiro et al., 2010, Critical Care 14:R182; Hetzel et al., 2005, Arterioscler. Thromb. Vasc. Biol. 25:1804-1809). Endothelial cells line, for example, the heart, blood and lymph vessels. Endothelial cells of blood vessels have both mechanical and functional properties, including for example, providing a barrier effect to the penetration of blood components into the vessel wall and have endocrine functions. Endothelial dysfunction may be characterized by a loss of barrier function and an infiltration of cellular material into the vascular wall and loss of physiological vascular tone. There may be a loss of nitric oxide mediated physiological vasodilation, increased endothelial adhesion and migration of leucocytes and macrophages into the subendothelial vascular wall. Numerous in vivo and in vitro techniques may be used to evaluate functional integrity of the endothelium, such as for example endothelial dependent vasomotion, assessed with RHI and endothelial mediated vasomotion, assessed using peripheral arterial tonometry (PAT) at the finger after upper-arm occlusion (endoPAT, Itamar). Endothelial cell function or dysfunction may be determined by measuring of one or more biomarkers. Endothelial cell functional biomarkers may include, for example, one or more of von Willebrand factor (vWF), ADAMTS13, angiopoietins (Ang)-1 and -2, endocan (*e.g.,* endothelial cell-specific molecule-1 (ESM-1)), selectins (*e.g.,* E-, P-, L-), syndecan 1 (SDC1, CD138), endothelial leukocyte adhesion molecule (E-selectin or ELAM-1), endothelin (ET-1), endothelin precursor peptide proET-1, VEGF, soluble VEGF-receptor-1 (Flt-1), PDGF, plasminogen activator inhibitor (PAI-1), urokinase PA (uPA) and fibrin degradation products (*e.g.,* X and Y fragments, D-dimers, D and E fragments, Bβ15-42). Biomarker levels may be measured by any of various methods known in the art, such as for example, by using commercially available enzyme-linked immunoabsorbent assay (ELISA) kits or electrochemiluminescence (ECL) platforms. In certain preferred embodiments, biomarkers are assessed in plasma or serum from the subject. An improvement may be an increase in a particular measured parameter, if an increase for such parameter is considered to indicate a beneficial change in endothelial cell function. An improvement may be a decrease in a particular measured parameter, if a decrease for such parameter is considered to indicate a beneficial change in endothelial cell function. In certain embodiments, an improvement to endothelial cell dysfunction may be at least about 2-fold, 3-fold, 4-fold, 5-fold, 6-fold, 7-fold, 8-fold, 9-fold, 10-fold, 15-fold, 20-fold, 30-fold, 40-fold, 50-fold, 100-fold, 500-fold, 1000-fold, or more change, as compared to the level measured prior to treatment/administration. Alternatively or in addition, an improvement to endothelial cell dysfunction may be at least about 5%, 10%, 20%, 30%, 40%, 50%, 60%, 70%, 80%, 90%, 95%, 99% or more change, as compared to the level measured prior to treatment/administration.

The invention is illustrated further by the following examples, which are not to be construed as limiting the invention in scope to the specific procedures described in them.

### EXAMPLES

### Example 1: Preservation of antibody function in plasma after pathogen inactivation

To determine the functional activity of antibody in plasma following treatment with a pathogen inactivation compound, a study was conducted to assess the binding affinity of IgG antibody in treated plasma to an example cognate antigen, tetanus toxoid. This antigen was selected due to the universal immunization to tetanus and therefore the presence of tetanus toxoid specific antibody in plasma donations. Paired samples of control plasma and plasma treated with a photo-activated amotosalen pathogen inactivation compound (INTERCEPT® Blood System; Irsch et al., 2011, Transfus. Med. Hemother. 38:19-31) from 60 apheresis plasma donations were tested by an ELISA assay for antibody to tetanus toxoid antigen using a commercially available Tetanus Toxoid IgG ELISA kit (GenWay Biotech, San Diego, CA), according to the manufacturer's instructions.

As shown in Table 1, no difference was observed between paired treated and untreated control plasma for IgG levels to tetanus toxoid antigen over a range of antibody levels. A range of IgG levels is expected across a population of blood donors, but regardless, the differences for paired treated plasma and control plasma were small and not significantly different, indicating that the pathogen inactivation process did not adversely affect antibody function.

**Table 1: IgG Antibody Levels (IU/mL) To Tetanus Toxoid in Paired INTERCEPT (Test) and Conventional Apheresis Plasma (Control) Prepared From Healthy Blood Donors**

| Site | Statistic | Antibody Concentration (IU/mL) | | |
|---|---|---|---|---|
| | | Test | Control | (Test - Control) |
| Overall | N | 83 | 83 | 83 |
| | Mean | 1.17 | 1.18 | 0.00 |
| | Median | 1.15 | 1.18 | -0.03 |
| | SD | 0.631 | 0.631 | 0.236 |
| | 95% CI of Mean | 1.03 to 1.31 | 1.04 to 1.31 | -0.06 to 0.05 |
| | Min to Max | 0.07 to 3.11 | 0.08 to 3.49 | -0.43 to 1.11 |

### Example 2: Evaluation of a plasma therapeutic for treatment of Ebola virus infection

The study is designed as a prospective, open label observational study to evaluate the safety and efficacy of amotosalen-UVA inactivated plasma (INTERCEPT® plasma) prepared from convalescent Ebola virus donors for passive immune therapy in subjects with acute Ebola virus infection. Efficacy is assessed by monitoring survival and the clinical status of treated subjects with respect to coagulopathy and also clearance of Ebola virus by using nucleic acid assays to measure pre- and post-treatment viral titers.

Plasma is collected from volunteer donors who have recovered from Ebola virus infection. The donor plasma is collected by apheresis donation (approximately 650 mL) and treated with the INTERCEPT® Blood System for plasma. The treated plasma is referred to as INTERCEPT EBOV plasma and the plasma is stored at -18 to -25 °C for up to 1 year. Donor recovery from Ebola virus infection is based on clinical status:
- Recovered from Ebola virus infection and discharged from hospital at least 30 days prior to donation
- Two negative assays for Ebola virus nucleic acid by RT-PCR
- Positive for IgG and IgM antibodies to Ebola virus antigens

For treatment, subjects are transfused with compatible INTERCEPT EBOV plasma, which has been assayed to determine the levels of Ebola virus specific antibodies. Each subject is infused with 15-20 ml/kg (e.g., at least 400-500 mL) INTERCEPT EBOV plasma, or an alternative dose that is consistent with the local standard of care (e.g., local standard for plasma infusion volume). Pediatric patients receive weight adjusted dosing of 10mL/kg. Plasma transfusion may be repeated, with no limitation to the number of transfusions or total dose of INTERCEPT EBOV plasma.

Blood samples are collected to assess Ebola virus titers by nucleic acid testing (NAT) approximately 4 hours after the completion of the study transfusion, or at a time designated by the clinical investigators. The primary measure of efficacy is the proportion of subjects who survive acute Ebola virus infection. Secondary measures of efficacy include assessments of subject hemostatic function pre- and post-plasma infusion (e.g., activated clotting time (ACT) test, coagulation tests, such as the prothrombin time (PT), activated partial thromboplastin time (aPTT), and/or thrombin time (TT), fibrinogen determination), and reduction/clearance of Ebola virus titers (assessed by NAT). Efficacy is also measured by time to clinical remission defined as absence of clinical symptoms indicative of Ebola virus disease and at least two negative Ebola virus nucleic acid assays at least 48 hours apart. In the event of death, time to death from initial diagnosis is determined.

Additional analyses of efficacy also include proportion of patients requiring hemodialysis after treatment with convalescent plasma, time to onset of hemodialysis, proportion of patients requiring assisted ventilation after INTERCEPT EBOV plasma transfusion, and time to assisted ventilation after INTERCEPT EBOV plasma transfusion. Analysis of markers of endothelial dysfunction, such as for example, von Willebrand Factor and Syndecan-1 levels are also evaluated.

### Example 3: Plasma therapeutic comprising a first and second plasma component

The study is designed as a prospective, open label observational study to evaluate the safety and efficacy of a plasma therapeutic comprising amotosalen-UVA inactivated plasma (INTERCEPT plasma) prepared from convalescent Ebola virus donors as a first component and non-immune plasma (from donors not previously infected with Ebola virus) as a second component, for passive immune therapy in subjects with acute Ebola virus infection. Efficacy is assessed by monitoring the clinical status of treated subjects with respect to coagulopathy and clearance of Ebola virus by using nucleic acid assays to measure pre- and post-treatment viral titers.

Plasma is collected from volunteer donors who have never been infected with Ebola virus and from donors who have recovered from Ebola virus infection. The donor plasma is collected by apheresis donation (approximately 650 mL) and treated with the INTERCEPT Blood System for plasma. The treated plasma from donors who have recovered from Ebola virus infection is referred to as INTERCEPT EBOV plasma and the Ebola-naïve donor plasma is referred to as INTERCEPT non-immune plasma. Both plasmas are stored at -18 to 25 °C for up to 1 year. Donor recovery from Ebola virus infection is based on clinical status as above.

Subjects are transfused with compatible INTERCEPT EBOV plasma, which has been assayed to determine the levels of Ebola virus specific antibodies, and INTERCEPT non-immune plasma. Each subject is infused with 15-20 ml/kg (e.g., at least 400-500 mL) INTERCEPT EBOV plasma and INTERCEPT non-immune plasma, or an alternative dose that is consistent with the local standard of care (e.g., local standard for plasma infusion volume). Pediatric patients receive weight adjusted dosing of 10mL/kg. Plasma transfusion may be repeated, with no limitation to the number of transfusions or total dose of INTERCEPT EBOV plasma. In the event of therapeutic plasma exchange with INTERCEPT non-immune plasma, the transfusion is at a dose of 40-60 mL/kg.

Blood samples are collected to assess Ebola virus titers by NAT approximately 4 hours after the completion of the study transfusion, or at a time designated by the clinical investigators. The primary measure of efficacy is the proportion of subjects who survive acute Ebola virus infection. Secondary measures of efficacy include assessments of subject hemostatic function pre- and post-plasma infusion, and reduction/clearance of Ebola virus titers (assessed by nucleic acid testing). Efficacy is also measured by time to clinical remission defined as absence of clinical symptoms indicative of Ebola virus disease and at least two negative Ebola virus nucleic acid assays at least 48 hours apart. In the event of death, time to death from initial diagnosis is determined.

Additional analyses of efficacy also include proportion of patients requiring hemodialysis after treatment with convalescent plasma, time to onset of hemodialysis, proportion of patients requiring assisted ventilation after plasma infusion, and time to assisted ventilation after plasma infusion. Analysis of markers of endothelial dysfunction, such as for example, von Willebrand Factor and Syndecan-1 levels are also evaluated.

### Example 4: Plasma therapeutic treatment with plasma exchange

The study is designed as a prospective, open label observational study to evaluate the safety and efficacy of a plasma therapeutic comprising amotosalen-UVA inactivated plasma (INTERCEPT plasma) prepared from convalescent Ebola virus donors as a first component and non-immune plasma (from donors not previously infected with Ebola virus) as a second component and used in a plasma exchange procedure, for passive immune therapy in subjects with acute Ebola virus infection. Efficacy is assessed by monitoring the clinical status of treated subjects with respect to coagulopathy and clearance of Ebola virus by using nucleic acid assays to measure pre- and post-treatment viral titers.

Plasma is collected from volunteer donors who have never been infected with Ebola virus and from donors who have recovered from Ebola virus infection. The donor plasma is collected by apheresis donation (approximately 650 mL) and treated with the INTERCEPT Blood System for plasma. The treated plasma from donors who have recovered from Ebola virus infection is referred to as INTERCEPT EBOV plasma and the Ebola-naïve donor plasma is referred to as INTERCEPT non-immune plasma. Both plasmas are stored at -18 to 25 °C for up to 1 year. Donor recovery from Ebola virus infection is based on clinical status as above.

Subjects are first subjected to therapeutic plasma exchange using INTERCEPT non-immune plasma at 60 mL/kg subject weight. Following the therapeutic plasma exchange, the subjects are transfused with compatible INTERCEPT EBOV plasma, which has been assayed to determine the levels of Ebola virus specific antibodies. Each subject is infused two times with 250 mL INTERCEPT EBOV plasma, or an alternative dose that is consistent with the local standard of care (e.g., local standard for plasma infusion volume). Additional INTERCEPT EBOV plasma infusions may be repeated, with no limitation to the number of transfusions or total dose of INTERCEPT EBOV plasma.

Blood samples are collected to assess Ebola virus titers by NAT approximately 4 hours after the completion of the study transfusion, or at a time designated by the clinical investigators. The primary measure of efficacy is the proportion of subjects who survive acute Ebola virus infection. Secondary measures of efficacy include assessments of subject hemostatic function pre- and post-plasma infusion, and reduction/clearance of Ebola virus titers (assessed by nucleic acid testing). Efficacy is also measured by time to clinical remission defined as absence of clinical symptoms indicative of Ebola virus disease and at least two negative Ebola virus nucleic acid assays at least 48 hours apart. In the event of death, time to death from initial diagnosis is determined.

Additional analyses of efficacy also include proportion of patients requiring hemodialysis after treatment with convalescent plasma, time to onset of hemodialysis, proportion of patients requiring assisted ventilation after plasma infusion, and time to assisted ventilation after plasma infusion. Analysis of markers of endothelial dysfunction, such as for example, von Willebrand Factor and Syndecan-1 levels are also evaluated.

The use of the terms "a" and "an" and "the" and similar referents (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Wherever an open-ended term is used to describe a feature or element, it is specifically contemplated that a closed-ended term can be used in place of the open-ended term without departing from the spirit and scope of the disclosure. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the description and does not pose a limitation on the scope of the description unless otherwise claimed. No language in the specification should be construed as indicating any non-claimed element as essential to the practice of the compositions and methods disclosed herein.

Preferred embodiments are described herein. Variations of those preferred embodiments may become apparent to those working in the art upon reading the foregoing description. It is expected that skilled artisans will be able to employ such variations as appropriate, and practice the compositions and methods described herein otherwise than as specifically described herein. Accordingly, the compositions and methods described herein include all modifications and equivalents of the subject matter recited in the claims appended hereto as permitted by applicable law. Moreover, any combination of the above-described elements in all possible variations thereof is encompassed by the description unless otherwise indicated herein or otherwise clearly contradicted by context.

## Claims

1. A first plasma component for use in a method of treating a subject suffering from a hemorrhagic fever virus infection in combination with a second plasma component, said method comprising administering to the subject a therapeutically effective amount of said first and said second plasma component, wherein the first plasma component is obtained from one or more plasma donors previously infected with the same type of hemorrhagic fever virus and the second plasma component is obtained from one or more plasma donors not previously infected with the same type of hemorrhagic fever virus; wherein the first plasma component or donor plasma obtained for the first plasma component is treated with a pathogen inactivation compound to inactivate pathogens; wherein the hemorrhagic fever virus is a bunyavirus, arenavirus, flavivirus or filovirus; and wherein optionally the filovirus is an Ebola virus or a Marburg virus

2. A first and a second plasma component for use in combination in a method of treating a subject suffering from a hemorrhagic fever virus infection, said method comprising administering to the subject a therapeutically effective amount of said first and said second plasma component, wherein the first plasma component is obtained from one or more plasma donors previously infected with the same type of hemorrhagic fever virus and the second plasma component is obtained from one or more plasma donors not previously infected with the same type of hemorrhagic fever virus; wherein the first plasma component or donor plasma obtained for the first plasma component is treated with a pathogen inactivation compound to inactivate pathogens; wherein the hemorrhagic fever virus is a bunyavirus, arenavirus, flavivirus or filovirus; and wherein optionally the filovirus is an Ebola virus or a Marburg virus.

3. The plasma for use according to claim 1 or claim 2, wherein the first plasma component is obtained from one donor, the second plasma component is obtained from one donor, or both the first plasma component is obtained from one donor and the second plasma component is obtained from one donor.

4. The plasma for use according to claim 1 or claim 2, wherein the first plasma component is obtained from at least two donors, the second plasma component is obtained from at least two donors, or both the first plasma component is obtained from at least two donors and the second plasma component is obtained from at least two donors.

5. The plasma for use according to any one of claims 1 to 4, wherein the first plasma component, the second plasma component, or both the first plasma component and second plasma component comprises donor plasma only of the same ABO blood group as the subject.

6. The plasma for use according to claim 4, wherein the first plasma component, the second plasma component, or both the first plasma component and second plasma component comprises donor plasma of more than one ABO blood group; wherein optionally the first plasma component the second plasma component, or both the first plasma component and second plasma component comprises donor plasma of blood group A and blood group B; and wherein optionally the second plasma component, or both the first plasma component and second plasma component does not contain donor plasma of blood group O.

7. The plasma for use according to claim 6, wherein the first plasma component is obtained from at least 3 donors and wherein the first plasma component comprises donor plasma of blood group A, blood group B and blood group AB; and/or wherein the second plasma component is obtained from at least 3 donors and wherein the second plasma component comprises donor plasma of blood group A, blood group B and blood group AB.

8. The plasma for use according to claim 1 or claim 2, wherein the first plasma component is obtained only from donors with no clinical symptoms of infection with the hemorrhagic fever virus at the time of donating the plasma for said first plasma component; and/or wherein the first plasma component is obtained only from donors with no detectable levels of the hemorrhagic fever virus in their blood at the time of donating the plasma for said first plasma component.

9. The plasma for use according to claim 1 or claim 2, wherein the first plasma component comprises about 10-90% of the total plasma volume administered and the second plasma component comprises the remainder of the total plasma volume administered; wherein optionally the first plasma component comprises about 10-50% of the total plasma volume administered and the second plasma component comprises the remainder of the total plasma volume administered.

10. The plasma for use according to claim 1 or claim 2, wherein the first plasma component, the second plasma component, or both the first plasma component and second plasma component are lyophilized for storage and reconstituted prior to administration of the plasma to the subject.

11. The plasma for use according to claim 1 or claim 2, wherein
(i) the first plasma component and the second plasma component are administered separately to the subject; wherein optionally the first plasma component and the second plasma component are administered within about 24 hours, within about 15 hours, within about 8 hours, within about 4 hours, within about 2 hours, or within about 1 hour of each other; wherein optionally the second plasma component is administered before the first plasma component and wherein optionally the second plasma component is administered as a plasma exchange; or
(ii) the first plasma component and the second plasma component are mixed prior to or during administration to the subject.

12. The plasma for use according to claim 1 or claim 2, wherein the plasma is administered to the subject in a volume of 300-1500 mL, or 10-60 mL/kg body weight of the subject or 15-20 mL/kg body weight of the subject.

13. The plasma for use according to claim 1 or claim 2, wherein the level of viral infection in the subject is reduced; and/or wherein the mortality associated with the virus infection is reduced; and/or wherein the number of co-morbidities or severity of morbidity is reduced; and/or wherein coagulopathy associated with the virus infection is reduced; and/or wherein endothelial cell dysfunction associated with the virus infection is decreased; and/or wherein endothelial barrier function is enhanced; and/or wherein time of hospitalization is reduced, time spent in ICU is reduced, total duration or frequency of dialysis is decreased, time on assisted ventilation is decreased, incidence of organ failure is reduced, or multifocal necrosis associated with virus infection is reduced.

14. The plasma for use according to claim 1 or claim 2, wherein the method further comprises treating the second plasma component or donor plasma obtained for the second plasma component with a pathogen inactivation compound to inactivate pathogens, if present: wherein optionally the pathogen inactivation compound is a photoactive pathogen inactivation compound selected from the group consisting of a psoralen, an isoalloxazine, an alloxazine, a phthalocyanine, a phenothiazine, a porphyrin, and merocyanine 540; wherein optionally the pathogen inactivation compound is a psoralen; wherein optionally the pathogen inactivation compound is amotosalen.

15. A plasma for use in a method of treating a hemorrhagic fever virus infection in a subject, comprising administering said plasma in an amount effective to treat coagulopathy or endothelial cell dysfunction, wherein the plasma comprises donor plasma obtained from one or more donors previously infected with or immunized against the same type of hemorrhagic fever virus, and wherein the plasma or donor plasma is treated with a pathogen inactivation compound to inactivate pathogens, if present; wherein optionally the hemorrhagic fever virus is a bunyavirus, arenavirus, flavivirus or filovirus; and wherein optionally the filovirus is an Ebola virus or a Marburg virus.

16. The plasma for use according to claim 15, wherein the donor plasma is obtained from at least two donors; wherein optionally the plasma comprises donor plasma only of the same ABO blood group as the subject; or wherein optionally the plasma comprises donor plasma of more than one ABO blood group: wherein the plasma comprises donor plasma of blood group A and blood group B; wherein optionally the plasma is obtained from at least 3 donors and wherein the plasma comprises donor plasma of blood group A, blood group B and blood group AB; and/or wherein optionally the plasma does not contain donor plasma of blood group O.

17. The plasma for use according to claim 15, wherein the donor plasma obtained from one or more donors previously infected with or immunized against the hemorrhagic fever virus is obtained only from donors with no clinical symptoms of infection with the virus at the time of donating the donor plasma; and/or wherein the donor plasma obtained from one or more donors previously infected with or immunized against the hemorrhagic fever virus is obtained only from donors with no detectable levels of the virus in their blood at the time of donating the donor plasma; wherein optionally no detectable levels of the virus in their blood is no detectable levels of the virus as determined by nucleic acid testing.

18. The plasma for use according to claim 15, wherein the plasma or donor plasma is lyophilized for storage and reconstituted prior to administration of the plasma to the subject; and/or wherein the plasma or donor plasma is a concentrated immunoglobulin preparation.

19. The plasma for use according to claim 15, wherein the donor plasma obtained from one or more donors previously infected with or immunized against the hemorrhagic fever virus is a first donor plasma in combination with a second donor plasma obtained from one or more donors not previously infected with or immunized against the same type of hemorrhagic fever virus, and wherein the second donor plasma is optionally treated with a pathogen inactivation compound to inactivate pathogens, if present: wherein optionally the second donor plasma is obtained from at least two donors; wherein optionally the second donor plasma comprises plasma only of the same ABO blood group as the subject; wherein optionally the second donor plasma comprises plasma of more than one ABO blood group; wherein optionally the second donor plasma comprises plasma of blood group A and blood group B; wherein optionally the second donor plasma is obtained from at least 3 donors and wherein the second donor plasma comprises plasma of blood group A, blood group B and blood group AB; and/or wherein optionally the second donor plasma does not contain plasma of blood group O.

20. The plasma for use according to claim 19, wherein the second donor plasma is lyophilized for storage and reconstituted prior to administration of the plasma to the subject or wherein the second donor plasma is a plasma cryoprecipitate.

21. The plasma for use according to claim 19, wherein the first donor plasma comprises about 10-90% of the total plasma volume administered and the second donor plasma comprises the remainder of the total plasma volume administered; wherein optionally the first donor plasma comprises about 10-50% of the total plasma volume administered and the second donor plasma comprises the remainder of the total plasma volume administered.

22. The plasma for use according to claim 19, wherein
(i) the first donor plasma and the second donor plasma are administered separately to the subject; wherein optionally the first donor plasma and the second donor plasma are administered within about 24 hours, within about 15 hours, within about 8 hours, within about 4 hours, within about 2 hours, or within about 1 hour of each other; wherein optionally the second donor plasma is administered before the first donor plasma; wherein optionally the second donor plasma is administered as a plasma exchange; or
(ii) the first donor plasma and the second donor plasma are mixed prior to or during administration to the subject.

23. The plasma for use according to claim 15 or claim 19, wherein the plasma is administered to the subject in a volume of 300-1500 mL, 10-60 mL/kg body weight of the subject or 15-20 mL/kg body weight of the subject.

24. The plasma for use according to claim 15 or claim 19, wherein the level of viral infection in the subject is reduced; and/or wherein the mortality associated with the virus infection is reduced; and/or wherein the number of co-morbidities or severity of morbidity is reduced; and/or wherein coagulopathy is reduced; and/or wherein endothelial cell dysfunction is decreased; and/or wherein endothelial barrier function is enhanced; and/or wherein time of hospitalization is reduced, time spent in ICU is reduced, total duration or frequency of dialysis is decreased, time on assisted ventilation is decreased, incidence of organ failure is reduced, or multifocal necrosis associated with virus infection is reduced.

25. The plasma for use according to claim 15 or claim 19, wherein the level of binding activity of hemorrhagic fever virus specific antibody in the donor plasma obtained from one or more donors previously infected with or immunized against the hemorrhagic fever virus, after treatment with the pathogen inactivation compound, is at least 80% of the level of binding activity of hemorrhagic fever virus specific antibody before treatment with the pathogen inactivation compound; and/or wherein the level of binding activity of hemorrhagic fever virus specific antibody in the plasma after treatment with the pathogen inactivation compound is at least 80% of the level of binding activity of hemorrhagic fever virus specific antibody before treatment with the pathogen inactivation compound.

## Patentansprüche

1. Erste Plasmakomponente zur Verwendung in einem Verfahren zur Behandlung eines Individuums, das an einer viralen hämorrhagischen Fieberinfektion leidet, in Kombination mit einer zweiten Plasmakomponente, wobei das Verfahren das Verabreichen einer therapeutisch wirksamen Menge der ersten und zweiten Plasmakomponente an das Individuum umfasst, wobei die erste Plasmakomponente von einem oder mehreren Plasmaspendern erhalten wurde, die zuvor mit der gleichen Art des hämorrhagischen Fiebervirus infiziert waren, und die zweite Plasmakomponente von einem oder mehreren Plasmaspendern erhalten wurde, die noch nicht zuvor mit der gleichen Art des hämorrhagischen Fiebervirus infiziert waren; wobei die erste Plasmakomponente oder das für die erste Plasmakomponente erhaltene Spenderplasma mit einer Pathogeninaktivierungsverbindung behandelt wird, um Pathogene zu inaktivieren; wobei das hämorrhagische Fiebervirus ein Bunyavirus, Arenavirus, Flavivirus oder Filovirus ist; und wobei das Filovirus gegebenenfalls ein Ebola-Virus oder ein Marburg-Virus ist.

2. Erste und zweite Plasmakomponente zur Verwendung in Kombination in einem Verfahren zur Behandlung eines Individuums, das an einer viralen hämorrhagischen Fieberinfektion leidet, wobei das Verfahren das Verabreichen einer therapeutisch wirksamen Menge der ersten und der zweiten Plasmakomponente an das Individuum umfasst, wobei die erste Plasmakomponente von einem oder mehreren Plasmaspendern erhalten wurde, die zuvor mit der gleichen Art des hämorrhagischen Fiebervirus infiziert waren, und die zweite Plasmakomponente von einem oder mehreren Plasmaspendern erhalten wurde, die noch nicht zuvor mit der gleichen Art des hämorrhagischen Fiebervirus infiziert waren; wobei die erste Plasmakomponente oder das für die erste Plasmakomponente erhaltene Spenderplasma mit einer Pathogeninaktivierungsverbindung behandelt wird, um Pathogene zu inaktivieren; wobei das hämorrhagische Fiebervirus ein Bunyavirus, Arenavirus, Flavivirus oder Filovirus ist; und wobei das Filovirus gegebenenfalls ein Ebola-Virus oder ein Marburg-Virus ist.

3. Plasma zur Verwendung nach Anspruch 1 oder 2, wobei die erste Plasmakomponente von einem Spender erhalten wurde, die zweite Plasmakomponente von einem Spender erhalten wurde oder sowohl die erste Plasmakomponente von einem Spender erhalten wurde als auch die zweite Plasmakomponente von einem Spender erhalten wurde.

4. Plasma zur Verwendung nach Anspruch 1 oder Anspruch 2, wobei die erste Plasmakomponente von zumindest zwei Spendern erhalten wurde, die zweite Plasmakomponente von zumindest zwei Spendern erhalten wurde oder sowohl die erste Plasmakomponente von zumindest zwei Spendern erhalten wurde als auch die zweite Plasmakomponente von zumindest zwei Spendern erhalten wurde.

5. Plasma zur Verwendung nach einem der Ansprüche 1 bis 4, wobei die erste Plasmakomponente, die zweite Plasmakomponente oder sowohl die erste Plasmakomponente als auch die zweite Plasmakomponente nur Spenderplasma der gleichen AB0-Blutgruppe wie das Individuum umfassen.

6. Plasma zur Verwendung nach Anspruch 4, wobei die erste Plasmakomponente, die zweite Plasmakomponente oder sowohl die erste Plasmakomponente als auch die zweite Plasmakomponente Spenderplasma von mehr als einer AB0-Blutgruppe umfassen; wobei die erste Plasmakomponente, die zweite Plasmakomponente oder sowohl die erste Plasmakomponente als auch die zweite Plasmakomponente gegebenenfalls Spenderplasma der Blutgruppe A oder Blutgruppe B umfassen; und wobei die zweite Plasmakomponente oder sowohl die erste Plasmakomponente als auch die zweite Plasmakomponente kein Spenderplasma der Blutgruppe 0 umfassen.

7. Plasma zur Verwendung nach Anspruch 6, wobei die erste Plasmakomponente von zumindest 3 Spendern erhalten wurde und wobei die erste Plasmakomponente Spenderplasma der Blutgruppe A, Blutgruppe B und Blutgruppe AB umfasst; und/oder wobei die zweite Plasmakomponente von zumindest 3 Spendern erhalten wurde und wobei die zweite Plasmakomponente Spenderplasma der Blutgruppe A, Blutgruppe B und Blutgruppe AB umfasst.

8. Plasma zur Verwendung nach Anspruch 1 oder Anspruch 2, wobei die erste Plasmakomponente nur von Spendern ohne klinische Symptome einer Infektion mit dem hämorrhagischen Fiebervirus zur Zeit der Spende des Plasmas für die erste Plasmakomponente erhalten wurde; und/oder wobei die erste Plasmakomponente nur von Spendern ohne detektierbare Ausmaße des hämorrhagischen Fiebervirus im Blut zur Zeit der Spende des Plasmas für die erste Plasmakomponente erhalten wurde.

9. Plasma zur Verwendung nach Anspruch 1 oder Anspruch 2, wobei die erste Plasmakomponente etwa 10 bis 90 % des insgesamt verabreichten Plasmavolumens umfasst und die zweite Plasmakomponente den Rest des insgesamt verabreichten Plasmavolumens umfasst; wobei die erste Plasmakomponente gegebenenfalls etwa 10 bis 50 % des insgesamt verabreichten Plasmavolumens umfasst und die zweite Plasmakomponente den Rest des insgesamt verabreichten Plasmavolumens umfasst.

10. Plasma zur Verwendung nach Anspruch 1 oder Anspruch 2, wobei die erste Plasmakomponente, die zweite Plasmakomponente oder sowohl die erste Plasmakomponente als auch die zweite Plasmakomponente zur Lagerung gefriergetrocknet sind und vor der Verabreichung des Plasmas an das Individuum rekonstituiert werden.

11. Plasma zur Verwendung nach Anspruch 1 oder Anspruch 2, wobei
(i) die erste Plasmakomponente und die zweite Plasmakomponente dem Individuum separat verabreicht werden; wobei die erste Plasmakomponente und die zweite Plasmakomponente gegebenenfalls innerhalb von etwa 24 Stunden, innerhalb von etwa 15 Stunden, innerhalb von etwa 8 Stunden, innerhalb von etwa 4 Stunden, innerhalb von etwa 2 Stunden oder innerhalb von etwa 1 Stunde voneinander verabreicht werden; wobei die zweite Plasmakomponente gegebenenfalls vor der ersten Plasmakomponente verabreicht wird und wobei die zweite Plasmakomponente gegebenenfalls als Plasmaaustausch verabreicht wird; oder
(ii) die erste Plasmakomponente und die zweite Plasmakomponente vor oder während der Verabreichung an das Individuum vermischt werden.

12. Plasma zur Verwendung nach Anspruch 1 oder Anspruch 2, wobei dem Individuum das Plasma in einem Volumen von 300 bis 1500 ml oder 10 bis 60 ml/kg Körpergewicht des Individuums oder 15 bis 20 ml/kg Körpergewicht des Individuums verabreicht wird.

13. Plasma zur Verwendung nach Anspruch 1 oder Anspruch 2, wobei das Ausmaß der Virusinfektion in dem Individuum reduziert wird; und/oder wobei die mit der Virusinfektion verbundene Sterblichkeit reduziert wird; und/oder wobei die Anzahl der Co-Morbiditäten oder die Schwere der Morbidität reduziert wird; und/oder wobei die mit der Virusinfektion verbundene Koagulopathie reduziert wird; und/oder wobei die mit der Virusinfektion verbundene Endothelzellendysfunktion vermindert wird; und/oder wobei die Endothelbarrierefunktion verstärkt wird; und/oder wobei die Dauer des Krankenhausaufenthalts reduziert wird, die auf der Intensivstation verbrachte Zeit reduziert wird, die Gesamtdauer oder Häufigkeit der Dialyse verringert wird, die Dauer der künstlichen Beatmung verringert wird, das Auftreten von Organversagen reduziert wird oder eine multifokale Nekrose aufgrund der Virusinfektion reduziert wird.

14. Plasma zur Verwendung nach Anspruch 1 oder Anspruch 2, wobei das Verfahren außerdem das Behandeln der zweiten Plasmakomponente oder des für die zweite Plasmakomponente erhaltenen Spenderplasmas mit einer Pathogeninaktivierungsverbindung umfasst, um Pathogene falls vorhanden zu inaktivieren; wobei die Pathogeninaktivierungsverbindung gegebenenfalls eine photoaktive Pathogeninaktivierungsverbindung ist, die aus der aus einem Psoralen, einem Isoalloxazin, einem Alloxazin, einem Phthalocyanin, einem Phenothiazin, einem Porphyrin und Merocyanin 540 bestehenden Gruppe ausgewählt ist; wobei die Pathogeninaktivierungsverbindung gegebenenfalls ein Psoralen ist; wobei die Pathogeninaktivierungsverbindung gegebenenfalls Amotosalen ist.

15. Plasma zur Verwendung in einem Verfahren zur Behandlung einer viralen hämorrhagischen Fieberinfektion bei einem Individuum, das das Verabreichen des Plasmas in einer zur Behandlung von Koagulopathie oder Endothelzellendysfunktion wirksamen Menge umfasst, wobei das Plasma Spenderplasma umfasst, das von einem oder mehreren Spendern, die zuvor mit der gleichen Art des hämorrhagischen Fiebervirus infiziert oder gegen dieses immunisiert wurden, erhalten wurde, und wobei das Plasma oder Spenderplasma mit einer Pathogeninaktivierungsverbindung behandelt wird, um Pathogene falls vorhanden zu inaktivieren; wobei das hämorrhagische Fiebervirus ein Bunyavirus, Arenavirus, Flavivirus oder Filovirus ist; und wobei das Filovirus gegebenenfalls ein Ebola-Virus oder ein Marburg-Virus ist.

16. Plasma zur Verwendung nach Anspruch 15, wobei das Spenderplasma von zumindest zwei Spendern erhalten wurde; wobei das Plasma gegebenenfalls nur Spenderplasma der gleichen AB0-Blutgruppe wie das Individuum umfasst; oder wobei das Plasma gegebenenfalls Spenderplasma von mehr als einer AB0-Blutgruppe umfasst; wobei das Plasma Spenderplasma der Blutgruppe A und Blutgruppe B umfasst; wobei das Plasma gegebenenfalls von zumindest 3 Spendern erhalten wurde und wobei das Plasma Spenderplasma der Blutgruppe A, Blutgruppe B und Blutgruppe AB umfasst; und/oder wobei das Plasma gegebenenfalls kein Spenderplasma der Blutgruppe 0 umfasst.

17. Plasma zur Verwendung nach Anspruch 15, wobei das Spenderplasma, das von einem oder mehreren Spendern, die zuvor mit dem hämorrhagischen Fiebervirus infiziert oder gegen dieses immunisiert wurden, erhalten wurde, nur von Spendern ohne klinische Symptome einer Infektion mit dem Virus zum Zeitpunkt der Spende des Spenderplasmas erhalten wurde; und/oder wobei das Spenderplasma, das von einem oder mehreren Spendern, die zuvor mit dem hämorrhagischen Fiebervirus infiziert oder gegen dieses immunisiert wurden, erhalten wurde, nur von Spendern ohne detektierbare Ausmaße des Virus im Blut zum Zeitpunkt der Spende des Spenderplasmas erhalten wurde; wobei keine detektierbaren Ausmaße des Virus im Blut gegebenenfalls mittels Nucleinsäuretestung bestimmt wird.

18. Plasma zur Verwendung nach Anspruch 15, wobei das Plasma oder Spenderplasma zur Lagerung gefriergetrocknet und vor der Verabreichung des Plasmas an das Individuum rekonstituiert wird; und/oder wobei das Plasma oder Spenderplasma ein konzentriertes Immunglobulinpräparat ist.

19. Plasma zur Verwendung nach Anspruch 15, wobei das Spenderplasma, das von einem oder mehreren Spendern, die zuvor mit dem hämorrhagischen Fiebervirus infiziert oder gegen dieses immunisiert wurden, erhalten wurde, ein erstes Spenderplasma in Kombination mit einem zweiten Spenderplasma, das von einem oder mehreren Spendern, die zuvor noch nicht mit der gleichen Art des hämorrhagischen Fiebervirus infiziert oder gegen dieses immunisiert wurden, erhalten wurde, ist und wobei das zweite Spenderplasma gegebenenfalls mit einer Pathogeninaktivierungsverbindung behandelt wird, um Pathogene falls vorhanden zu inaktivieren; wobei das zweite Spenderplasma gegebenenfalls von zumindest zwei Spendern erhalten wurde; wobei das zweite Spenderplasma gegebenenfalls nur Plasma der gleichen AB0-Blutgruppe wie das Individuum umfasst; wobei das zweite Spenderplasma gegebenenfalls Plasma von mehr als einer AB0-Blutgruppe umfasst; wobei das zweite Spenderplasma gegebenenfalls Plasma der Blutgruppe A und Blutgruppe B umfasst; wobei das zweite Spenderplasma gegebenenfalls von zumindest 3 Spendern erhalten wurde und wobei das zweite Spenderplasma Plasma der Blutgruppe A, Blutgruppe B und Blutgruppe AB umfasst; und/oder wobei das zweite Spenderplasma gegebenenfalls kein Plasma der Blutgruppe 0 enthält.

20. Plasma zur Verwendung nach Anspruch 19, wobei das zweite Spenderplasma zur Lagerung gefriergetrocknet und vor der Verabreichung des Plasmas an das Individuum rekonstituiert wird oder wobei das zweite Spenderplasma ein Plasmakryopräzipitat ist.

21. Plasma zur Verwendung nach Anspruch 19, wobei das erste Spenderplasma etwa 10 bis 90 % des insgesamt verabreichten Plasmavolumens und das zweite Spenderplasma den Rest des insgesamt verabreichten Plasmavolumens umfasst; wobei das erste Spenderplasma gegebenenfalls etwa 10 bis 50 % des insgesamt verabreichten Plasmavolumens und das zweite Spenderplasma den Rest des insgesamt verabreichten Plasmavolumens umfasst.

22. Plasma zur Verwendung nach Anspruch 19, wobei
(i) das erste Spenderplasma und das zweite Spenderplasma dem Individuum separat verabreicht werden; wobei das erste Spenderplasma und das zweite Spenderplasma gegebenenfalls innerhalb von etwa 24 Stunden, innerhalb von etwa 15 Stunden, innerhalb von etwa 8 Stunden, innerhalb von etwa 4 Stunden, innerhalb von etwa 2 Stunden oder innerhalb von etwa 1 Stunde voneinander verabreicht werden; wobei das zweite Spenderplasma gegebenenfalls vor dem ersten Spenderplasma verabreicht wird; wobei das zweite Spenderplasma gegebenenfalls als Plasmaaustausch verabreicht wird; oder
(ii) das erste Spenderplasma und das zweite Spenderplasma vor oder während der Verabreichung an das Individuum vermischt werden.

23. Plasma zur Verwendung nach Anspruch 15 oder Anspruch 19, wobei dem Individuum das Plasma in einem Volumen von 300 bis 1500 ml, 10 bis 60 ml/kg Körpergewicht des Individuums oder 15 bis 20 ml/kg Körpergewicht des Individuums verabreicht wird.

24. Plasma zur Verwendung nach Anspruch 15 oder Anspruch 19, wobei das Ausmaß der Virusinfektion in dem Individuum reduziert wird; und/oder wobei die mit der Virusinfektion verbundene Sterblichkeit reduziert wird; und/oder wobei die Anzahl der Co-Morbiditäten oder die Schwere der Morbidität reduziert wird; und/oder wobei die Koagulopathie reduziert wird; und/oder wobei die Endothelzellendysfunktion vermindert wird; und/oder wobei die Endothelbarrierefunktion verstärkt wird; und/oder wobei die Dauer des Krankenhausaufenthalts reduziert wird, die auf der Intensivstation verbrachte Zeit reduziert wird, die Gesamtdauer oder Häufigkeit der Dialyse verringert wird, die Dauer der künstlichen Beatmung verringert wird, das Auftreten von Organversagen reduziert wird oder eine multifokale Nekrose aufgrund der Virusinfektion reduziert wird.

25. Plasma zur Verwendung nach Anspruch 15 oder Anspruch 19, wobei das Ausmaß der Bindungsaktivität des für das hämorrhagische Fiebervirus spezifischen Antikörpers in dem Spenderplasma, das von einem oder mehreren Spendern, die zuvor mit dem hämorrhagischen Fiebervirus infiziert oder gegen dieses immunisiert wurden, erhalten wurde, nach der Behandlung mit der Pathogeninaktivierungsverbindung zumindest 80 % des Ausmaßes der Bindungsaktivität des für das hämorrhagische Fiebervirus spezifischen Antikörpers vor der Behandlung mit der Pathogeninaktivierungsverbindung beträgt; und/oder wobei das Ausmaß der Bindungsaktivität des für das hämorrhagische Fiebervirus spezifischen Antikörpers in dem Plasma nach der Behandlung mit der Pathogenaktivierungsverbindung zumindest 80 % des Ausmaßes der Bindungsaktivität des für das hämorrhagische Fiebervirus spezifischen Antikörpers vor der Behandlung mit der Pathogeninaktivierungsverbindung beträgt.

## Revendications

1. Premier composant de plasma destiné à être utilisé dans un procédé de traitement d'un sujet souffrant d'une infection par un virus de la fièvre hémorragique en combinaison avec un second composant de plasma, ledit procédé comprenant l'administration au sujet d'une quantité thérapeutiquement efficace dudit premier et dudit second composant de plasma, dans lequel le premier composant de plasma est obtenu à partir d'un ou de plusieurs donneurs de plasma précédemment infectés par le même type de virus de la fièvre hémorragique et le second composant de plasma est obtenu à partir d'un ou de plusieurs donneurs de plasma non précédemment infectés par le même type de virus de la fièvre hémorragique ; dans lequel le premier composant de plasma ou le plasma de donneur obtenu pour le premier composant de plasma est traité avec un composé d'inactivation des agents pathogènes afin d'inactiver les agents pathogènes ; dans lequel le virus de la fièvre hémorragique est un bunyavirus, un arénavirus, un flavivirus ou un filovirus ; et dans lequel facultativement le filovirus est un virus Ebola ou un virus Marburg.

2. Premier et second composants de plasma destinés à être utilisés en combinaison dans un procédé de traitement d'un sujet souffrant d'une infection par un virus de la fièvre hémorragique, ledit procédé comprenant l'administration au sujet d'une quantité thérapeutiquement efficace dudit premier et dudit second composant de plasma, dans lequel le premier composant de plasma est obtenu à partir d'un ou de plusieurs donneurs de plasma précédemment infectés par le même type de virus de la fièvre hémorragique et le second composant de plasma est obtenu à partir d'un ou de plusieurs donneurs de plasma non précédemment infectés par le même type de virus de la fièvre hémorragique ; dans lequel le premier composant de plasma ou le plasma de donneur obtenu pour le premier composant de plasma est traité avec un composé d'inactivation des agents pathogènes afin d'inactiver les agents pathogènes ; dans lequel le virus de la fièvre hémorragique est un bunyavirus, un arénavirus, un flavivirus ou un filovirus ; et dans lequel facultativement le filovirus est un virus Ebola ou un virus Marburg.

3. Plasma destiné à être utilisé selon la revendication 1 ou la revendication 2, dans lequel le premier composant de plasma est obtenu à partir d'un seul donneur, le second composant de plasma est obtenu à partir d'un seul donneur, ou à la fois le premier composant de plasma est obtenu à partir d'un seul donneur et le second composant de plasma est obtenu à partir d'un seul donneur.

4. Plasma destiné à être utilisé selon la revendication 1 ou la revendication 2, dans lequel le premier composant de plasma est obtenu à partir d'au moins deux donneurs, le second composant de plasma est obtenu à partir d'au moins deux donneurs, ou à la fois le premier composant de plasma est obtenu à partir d'au moins deux donneurs et le second composant de plasma est obtenu à partir d'au moins deux donneurs.

5. Plasma destiné à être utilisé selon l'une quelconque des revendications 1 à 4, dans lequel le premier composant de plasma, le second composant de plasma, ou à la fois le premier composant de plasma et le second composant de plasma comprennent du plasma de donneur uniquement du même groupe sanguin ABO que celui du sujet.

6. Plasma destiné à être utilisé selon la revendication 4, dans lequel le premier composant de plasma, le second composant de plasma, ou à la fois le premier composant de plasma et le second composant de plasma comprennent du plasma de donneur de plus d'un seul groupe sanguin ABO ; dans lequel facultativement le premier composant de plasma, le second composant de plasma, ou à la fois le premier composant de plasma et le second composant de plasma comprennent du plasma de donneur du groupe sanguin A et du groupe sanguin B ; et dans lequel facultativement le second composant de plasma, ou à la fois le premier composant de plasma et le second composant de plasma ne contiennent pas de plasma de donneur du groupe sanguin O.

7. Plasma destiné à être utilisé selon la revendication 6, dans lequel le premier composant de plasma est obtenu à partir d'au moins 3 donneurs et dans lequel le premier composant de plasma comprend du plasma de donneur du groupe sanguin A, du groupe sanguin B et du groupe sanguin AB ; et/ou dans lequel le second composant de plasma est obtenu à partir d'au moins 3 donneurs et dans lequel le second composant de plasma comprend du plasma de donneur du groupe sanguin A, du groupe sanguin B et du groupe sanguin AB.

8. Plasma destiné à être utilisé selon la revendication 1 ou la revendication 2, dans lequel le premier composant de plasma est obtenu uniquement à partir de donneurs qui ne présentent aucun symptôme clinique d'infection par le virus de la fièvre hémorragique au moment du don de plasma pour ledit premier composant de plasma ; et/ou dans lequel le premier composant de plasma est obtenu uniquement à partir de donneurs qui ne présentent aucun taux détectable du virus de la fièvre hémorragique dans leur sang au moment du don de plasma pour ledit premier composant de plasma.

9. Plasma destiné à être utilisé selon la revendication 1 ou la revendication 2, dans lequel le premier composant de plasma comprend environ 10-90 % du volume de plasma total administré et le second composant de plasma comprend le reste du volume de plasma total administré ; dans lequel facultativement le premier composant de plasma comprend environ 10-50 % du volume de plasma total administré et le second composant de plasma comprend le reste du volume de plasma total administré.

10. Plasma destiné à être utilisé selon la revendication 1 ou la revendication 2, dans lequel le premier composant de plasma, le second composant de plasma, ou à la fois le premier composant de plasma et le second composant de plasma sont lyophilisés pour le stockage et reconstitués avant l'administration du plasma au sujet.

11. Plasma destiné à être utilisé selon la revendication 1 ou la revendication 2, dans lequel
(i) le premier composant de plasma et le second composant de plasma sont administrés séparément au sujet ; dans lequel facultativement le premier composant de plasma et le second composant de plasma sont administrés à environ 24 heures, à environ 15 heures, à environ 8 heures, à environ 4 heures, à environ 2 heures, ou à environ 1 heure l'un après l'autre ; dans lequel facultativement le second composant de plasma est administré avant le premier composant de plasma et dans lequel facultativement le second composant de plasma est administré en tant qu'échange plasmatique ; ou
(ii) le premier composant de plasma et le second composant de plasma sont mélangés avant ou pendant l'administration au sujet.

12. Plasma destiné à être utilisé selon la revendication 1 ou la revendication 2, dans lequel le plasma est administré au sujet en un volume de 300-1 500 ml, ou 10-60 ml/kg de poids corporel du sujet ou 15-20 ml/kg de poids corporel du sujet.

13. Plasma destiné à être utilisé selon la revendication 1 ou la revendication 2, dans lequel le taux d'infection virale chez le sujet est réduit ; et/ou dans lequel la mortalité associée à l'infection par le virus est réduite ; et/ou dans lequel le nombre de comorbidités ou la sévérité de la morbidité est réduit(e) ; et/ou dans lequel la coagulopathie associée à l'infection par le virus est réduite ; et/ou dans lequel la dysfonction des cellules endothéliales associée à l'infection par le virus est réduite ; et/ou dans lequel la fonction de la barrière endothéliale est améliorée ; et/ou dans lequel le temps d'hospitalisation est réduit, le temps passé en USI est réduit, la durée totale ou la fréquence de la dialyse est réduite, le temps sous ventilation assistée est réduit, l'incidence d'une défaillance viscérale est réduite, ou la nécrose multifocale associée à l'infection par le virus est réduite.

14. Plasma destiné à être utilisé selon la revendication 1 ou la revendication 2, dans lequel le procédé comprend en outre le traitement du second composant de plasma ou du plasma de donneur obtenu pour le second composant de plasma avec un composé d'inactivation des agents pathogènes afin d'inactiver les agents pathogènes, si présents : dans lequel facultativement le composé d'inactivation des agents pathogènes est un composé d'inactivation des agents pathogènes photoactif sélectionné dans le groupe consistant en un psoralène, une isoalloxazine, une alloxazine, une phtalocyanine, une phénothiazine, une porphyrine, et la mérocyanine 540 ; dans lequel facultativement le composé d'inactivation des agents pathogènes est un psoralène ; dans lequel facultativement le composé d'inactivation des agents pathogènes est l'amotosalène.

15. Plasma destiné à être utilisé dans un procédé de traitement d'une infection par un virus de la fièvre hémorragique chez un sujet, comprenant l'administration dudit plasma en une quantité efficace pour traiter la coagulopathie ou la dysfonction des cellules endothéliales, dans lequel le plasma comprend du plasma de donneur obtenu à partir d'un ou de plusieurs donneurs précédemment infectés par ou immunisés contre le même type de virus de la fièvre hémorragique ; dans lequel le plasma ou le plasma de donneur est traité avec un composé d'inactivation des agents pathogènes afin d'inactiver les agents pathogènes, si présents ; dans lequel facultativement le virus de la fièvre hémorragique est un bunyavirus, un arénavirus, un flavivirus ou un filovirus ; et dans lequel facultativement le filovirus est un virus Ebola ou un virus Marburg.

16. Plasma destiné à être utilisé selon la revendication 15, dans lequel le plasma de donneur est obtenu à partir d'au moins deux donneurs ; dans lequel facultativement le plasma comprend du plasma de donneur uniquement du même groupe sanguin ABO que celui du sujet ; ou dans lequel facultativement le plasma comprend du plasma de donneur de plus d'un seul groupe sanguin ABO : dans lequel le plasma comprend du plasma de donneur du groupe sanguin A et du groupe sanguin B ; dans lequel facultativement le plasma est obtenu à partir d'au moins 3 donneurs et dans lequel le plasma comprend du plasma de donneur du groupe sanguin A, du groupe sanguin B et du groupe sanguin AB ; et/ou dans lequel facultativement le plasma ne contient pas de plasma de donneur du groupe sanguin O.

17. Plasma destiné à être utilisé selon la revendication 15, dans lequel le plasma de donneur obtenu à partir d'un ou de plusieurs donneurs précédemment infectés par ou immunisés contre le virus de la fièvre hémorragique est obtenu uniquement à partir de donneurs qui ne présentent aucun symptôme clinique d'infection par le virus au moment du don de plasma de donneur ; et/ou dans lequel le plasma de donneur obtenu à partir d'un ou de plusieurs donneurs précédemment infectés par ou immunisés contre le virus de la fièvre hémorragique est obtenu uniquement à partir de donneurs qui ne présentent aucun taux détectable du virus dans leur sang au moment du don de plasma de donneur ; dans lequel facultativement aucun taux détectable du virus dans leur sang signifie aucun taux détectable du virus tel que déterminé par un test des acides nucléiques.

18. Plasma destiné à être utilisé selon la revendication 15, dans lequel le plasma ou le plasma de donneur est lyophilisé pour le stockage et reconstitué avant l'administration du plasma au sujet ; et/ou dans lequel le plasma ou le plasma de donneur est une préparation d'immunoglobulines concentrée.

19. Plasma destiné à être utilisé selon la revendication 15, dans lequel le plasma de donneur obtenu à partir d'un ou de plusieurs donneurs précédemment infectés par ou immunisés contre le virus de la fièvre hémorragique est un premier plasma de donneur en combinaison avec un second plasma de donneur obtenu à partir d'un ou de plusieurs donneurs non précédemment infectés par ou immunisés contre le même type de virus de la fièvre hémorragique, et dans lequel le second plasma de donneur est facultativement traité avec un composé d'inactivation des agents pathogènes afin d'inactiver les agents pathogènes, si présents : dans lequel facultativement le second plasma de donneur est obtenu à partir d'au moins deux donneurs ; dans lequel facultativement le second plasma de donneur comprend du plasma uniquement du même groupe sanguin ABO que celui du sujet ; dans lequel facultativement le second plasma de donneur comprend du plasma de plus d'un seul groupe sanguin ABO ; dans lequel facultativement le second plasma de donneur comprend du plasma du groupe sanguin A et du groupe sanguin B ; dans lequel facultativement le second plasma de donneur est obtenu à partir d'au moins 3 donneurs et dans lequel le second plasma de donneur comprend du plasma du groupe sanguin A, du groupe sanguin B et du groupe sanguin AB ; et/ou dans lequel facultativement le second plasma de donneur ne contient pas de plasma du groupe sanguin O.

20. Plasma destiné à être utilisé selon la revendication 19, dans lequel le second plasma de donneur est lyophilisé pour le stockage et reconstitué avant l'administration du plasma au sujet ou dans lequel le second plasma de donneur est un cryoprécipité de plasma.

21. Plasma destiné à être utilisé selon la revendication 19, dans lequel le premier plasma de donneur comprend environ 10-90 % du volume de plasma total administré et le second plasma de donneur comprend le reste du volume de plasma total administré ; dans lequel facultativement le premier plasma de donneur comprend environ 10-50 % du volume de plasma total administré et le second plasma de donneur comprend le reste du volume de plasma total administré.

22. Plasma destiné à être utilisé selon la revendication 19, dans lequel
(i) le premier plasma de donneur et le second plasma de donneur sont administrés séparément au sujet ; dans lequel facultativement le premier plasma de donneur et le second plasma de donneur sont administrés à environ 24 heures, à environ 15 heures, à environ 8 heures, à environ 4 heures, à environ 2 heures, ou à environ 1 heure l'un après l'autre ; dans lequel facultativement le second plasma de donneur est administré avant le premier plasma de donneur ; dans lequel facultativement le second plasma de donneur est administré en tant qu'échange plasmatique ; ou
(ii) le premier plasma de donneur et le second plasma de donneur sont mélangés avant ou pendant l'administration au sujet.

23. Plasma destiné à être utilisé selon la revendication 15 ou la revendication 19, dans lequel le plasma est administré au sujet en un volume de 300-1 500 ml, 10-60 ml/kg de poids corporel du sujet ou 15-20 ml/kg de poids corporel du sujet.

24. Plasma destiné à être utilisé selon la revendication 15 ou la revendication 19, dans lequel le taux d'infection virale chez le sujet est réduit ; et/ou dans lequel la mortalité associée à l'infection par le virus est réduite ; et/ou dans lequel le nombre de comorbidités ou la sévérité de la morbidité est réduit(e) ; et/ou dans lequel la coagulopathie est réduite ; et/ou dans lequel la dysfonction des cellules endothéliales est réduite ; et/ou dans lequel la fonction de la barrière endothéliale est améliorée ; et/ou dans lequel le temps d'hospitalisation est réduit, le temps passé en USI est réduit, la durée totale ou la fréquence de la dialyse est réduite, le temps sous ventilation assistée est réduit, l'incidence d'une défaillance viscérale est réduite, ou la nécrose multifocale associée à l'infection par le virus est réduite.

25. Plasma destiné à être utilisé selon la revendication 15 ou la revendication 19, dans lequel le taux d'activité de liaison d'un anticorps spécifique au virus de la fièvre hémorragique dans le plasma de donneur obtenu à partir d'un ou de plusieurs donneurs précédemment infectés par ou immunisés contre le virus de la fièvre hémorragique, après un traitement avec le composé d'inactivation des agents pathogènes, est au moins 80 % du taux d'activité de liaison d'un anticorps spécifique au virus de la fièvre hémorragique avant le traitement avec le composé d'inactivation des agents pathogènes ; et/ou dans lequel le taux d'activité de liaison d'un anticorps spécifique au virus de la fièvre hémorragique dans le plasma après un traitement avec le composé d'inactivation des agents pathogènes est au moins 80 % du taux d'activité de liaison d'un anticorps spécifique au virus de la fièvre hémorragique avant le traitement avec le composé d'inactivation des agents pathogènes.
